# EUROPEAN PATENT APPLICATION

(11) **EP 4 151 660 A1**
(43) Date of publication of application: **22.03.2023**
(21) Application number: 21809833.3
(22) Date of filing: 21.05.2021
(51) Int. Cl.: C07K 19/00, C12N 15/63, C12N 5/10, A61K 39/00, A61P 35/00

(54) **FUSION PROTEIN FOR REVERSING TUMOR MICROENVIRONMENT AND USE THEREOF**

(30) Priority: 22.05.2020 CN 202010441127
(71) Applicant: Chongqing Precision Biotech Company Limited, Chongqing 400038 (CN); Chongqing Precision Biological Industrial Technology Research Institute Co., Ltd., Chongqing 400038 (CN)
(72) Inventor: XU, Yanmin, Chongqing 400039 (CN); MEI, Endian, Chongqing 400039 (CN); ZHAO, Yongchun, Chongqing 400039 (CN); SHAN, Juanjuan, Chongqing 400039 (CN); QI, Yanan, Chongqing 400039 (CN); ZHAO, WenXu, Chongqing 400039 (CN); CHEN, Jun, Chongqing 400039 (CN); HUANG, Xia, Chongqing 400039 (CN)
(74) Representative: karo IP
(86) International application number: PCT/CN2021/095107
(87) International publication number: WO 2021/233411

(57) **Abstract**

The present application belongs to the technical field of immunotherapy, and specifically relates to a fusion protein for reversing a tumor microenvironment, a tumor immunosuppressive and resistant CAR and an expression vector, an immune cell and the use. The fusion protein is combined with different CAR structures of different targets, such as CEA, CD19, PSCA and BCMA, to form the tumor immunosuppressive and resistant CAR, or is used in combination with same to target and kill CD47 positive tumor cells; and the tumor immunosuppressive and resistant CAR and immune cells break through the influences of inhibitory signals on the function of CAR-T, which realizes the effectiveness of CAR-T treatment and can also ensure a certain degree of safety at the same time.

## Description

### FIELD OF THE INVENTION

The invention belongs to the technical field of immunotherapy, and specifically relates to a fusion protein for reversing the tumor microenvironment, a novel tumor immunosuppression-resistant CAR, an expression vector, immune cells and applications thereof.

### BACKGROUND OF THE INVENTION

The full name of CAR is chimeric antigen receptor, and CAR-T is chimeric antigen receptor T cell (i.e. T lymphocyte expressing the chimeric antigen receptor (CAR)). At present, CAR-T therapy has made breakthroughs in hematological tumors. However, the efficacy for solid tumors is not as good as that for hematological tumors. On the one hand, it is difficult for CAR-T to enter solid tumors. On the other hand, even if CAR-T cells enter into solid tumors, they cannot function normally due to the tumor microenvironment. These all affect the efficacy of CAR-T cells in the treatment of solid tumors; the tumor microenvironment is closely related to the occurrence, growth and metastasis of tumors. In recent years, clinical studies have shown that the tumor microenvironment, especially the immune microenvironment of solid tumors, has a significant impact on the efficacy and prognosis of immunotherapy (including immune cell therapy). Therefore, in the treatment of solid tumors with CAR-T cells, it is essential to get rid of tumor microenvironment inhibition, that is, to implement tumor microenvironment resistance.

As a new immune checkpoint following PD-1/PD-L1 and CTLA-4, CD47 inhibits the progress of innate immunity by transmitting a "don't eat me" signal to macrophages. At present, it is reported in the literature that CAR-T is prepared with ScFv targeting CD47 for tumor treatment. CD47 can contribute to the immune microenvironment for tumor escape. However, due to the heterogeneity of tumors, targeting CD47 alone has limited efficacy, moreover, because of its strong affinity, exogenous ScFv may cause CAR-T cells to be over activated, poor in survival and non-specific, leading to potential safety hazards. Therefore, it is very important to adopt a safer and more effective approach to identify CD47 and disrupt the escape signal of tumor microenvironment.

Signal regulatory protein alpha (SIRPα) is one of the ligands of CD47, which can bind to CD47 and inhibit the phagocytosis of macrophages. At present, most studies on CD47 focus on SIRPα. SIRPγ can also bind to CD47. It exists on the surface of T cells, and the extracellular region of which consists of a V domain and two C1 domains. There is no intracellular signal, and only one-way signal is transmitted through CD47. At present, few researchers have modified SIRPγ, which we believe is a more suitable ligand for tumor microenvironment disruption against CD47. Therefore, we selected the extracellular domain of SIRPγ protein for engineering design, and further designed a tumor immunosuppression-resistant CAR.

### SUMMARY OF THE INVENTION

The purpose of the present application is to provide a fusion protein for reversing the tumor microenvironment, an expression vector and an immune cell comprising the protein, and the fusion protein can reverse the tumor microenvironment, and tumor cells can be targeted and killed.

In order to achieve the above purpose, the present application adopts the following solution.

The extracellular domain of the SIRPγ protein is modified and designed to obtain a fusion protein that disrupts the inhibitory signal in tumor tissue and reverse the tumor microenvironment.

The fusion protein is an SIRPγ fusion protein, and the structure of the SIRPγ fusion protein comprises an extracellular domain, a transmembrane domain and an intracellular signaling domain.

Further, the transmembrane domain is derived from the human CD28 transmembrane domain or the human CD8 transmembrane domain.

Further, the amino acid sequence of the transmembrane domain is shown in SEQ ID NO:7 or SEQ ID NO:8.

Preferably, the intracellular signaling domain is derived from CD28, and the sequence of the intracellular signaling domain is shown in SEQ ID NO:9 or SEQ ID NO:38.

Further, the structure of the SIRPγ fusion protein is SIRPy-CD28TM-CD28 or SIRPγ-CD8TM-4-1BB.

Further, the amino acid sequence of the extracellular domain of SIRPγ is shown in SEQ ID NO: 1 or is a functional variant thereof.

Further, the amino acid sequence of the SIRPγ fusion protein SIRPy-CD28TM-CD28 is shown in SEQ ID NO: 2 or is a functional variant thereof.

Further, the amino acid sequence of the SIRPγ fusion protein SIRPγ-CD8TM-4-1BB is shown in SEQ ID NO: 3 or is a functional variant thereof.

Further, the nucleotide sequence of the SIRPγ fusion protein SIRPy-CD28TM-CD28 is shown in SEQ ID NO: 13.

Further, the nucleotide sequence of the SIRPγ fusion protein SIRPγ-CD8TM-4-1BB is shown in SEQ ID NO: 14.

Further, it comprises immune cells comprising the expression vector.

Further, the immune cells are T cells, T cell precursors or NK cells.

Specifically, the T cells can be αβT cells or γδT cells; the γδT cells are a unique subset of T cells that comprise receptor γ and δ chains on the surface, accounting for 0.5-5% of all T lymphocytes. Cell populations were first discovered in 1987. Although it has a γδ TCR, its recognition of antigens or ligands is not MHC-restricted, unlike traditional αβ T cells. At present, many patents such as CN107810267A and CN107771215A have disclosed the use of γδT cells in cell therapies and CAR-T cell therapies.

Specifically, while designing the SIRPγ fusion protein, PD-1 fusion proteins PD-1-28TM-28 and PD-1-8TM-BB are also designed.

Specifically, when the designed SIRPγ fusion protein is used in combination with CAR1, it is expressed either alone or co-expressed with CAR1 to play a role. And the SIRPγ fusion protein can also be used alone in immunotherapy.

Specifically, in the present application, the inventors believe that SIRPγ protein is a more suitable ligand for tumor microenvironment disruption against CD47. Therefore, the extracellular domain of SIRPγ protein is selected to be modified and designed, and a tumor immunosuppression-resistant CAR is further designed; therefore, the present application creatively combines the designed and constructed hypoxia inducible promoter with CAR-T cell technology, and combines hypoxia inducible promoter and SIRPγ protein with CAR-T cell technology, and apply them to tumor immunotherapy, that is, the traditional CAR structure is further modified to improve the therapeutic efficacy of the CAR-T on solid tumors and the safety of the CAR-T.

Further, the immune cell also comprises a chimeric antigen receptor structure that recognizes a tumor antigen, and the chimeric antigen receptor comprises an extracellular domain, a hinge region, a transmembrane domain and an intracellular signaling domain that recognizes a tumor antigen, wherein the tumor antigen include but is not limited to PSCA, PSMA, CD19, BCMA, CD123, CD20, CD22, CEA, EGFR, EGFRVIII, GPC3, 5T4, CD33, Her2, GD2, CD70, CLL-1, Trop2, CD47, GPC3, CLND18.2 , CD133, CS1, CD155, CD30, ROR1, MUC1, IL13RAα2 or mesothelin that can be used as antigenic molecules for tumor-targeted recognition.

The purpose of the present application is to further provide a novel tumor immunosuppression-resistant CAR, as well as an expression vector and an immune cell comprising the CAR. The novel tumor immunosuppression-resistant CAR and the immune cell disrupt the effect of inhibitory signals on the function of CAR-T in tumor tissue, thereby realizing the effectiveness of CAR-T therapy and ensuring a certain degree of safety.

For achieving the above object, the present application adopts the following solution:
The novel tumor immunosuppression-resistant CAR comprises the fusion protein for reversing the tumor microenvironment described in purpose 1 and CAR1, and the CAR1 comprises an extracellular domain recognizing a tumor antigen, a hinge region, a transmembrane domain and an intracellular signaling domain.

Further, the fusion protein is linked to the CAR1 through a polycistronic structure, and the polycistronic structure is a self-cleaving polypeptide or an internal ribosome entry site IRES, and the self-cleaving polypeptide is T2A, P2A, E2A or F2A.

Further, the CAR structure is an ScFv-hinge-TM-CD3ζ-self-cleaving peptide-SIRPy fusion protein or an ScFv-hinge-TM-4-1BB-CD3ζ-self-cleaving peptide-SIRPy fusion protein.

Further, the CAR structure is an ScFv-hinge-TM-CD3ζ-self-cleaving peptide-SIRPγ-CD28TM-CD28 or an ScFv-hinge-TM-4-1BB-CD3ζ-self-cleaving peptide-SIRPy-CD28TM-CD28.

Further, the CAR structure can be a conventional first-generation, second-generation, or third-generation structure of the CAR, or can be an improved dual-CAR structure, a regulable CAR structure (such as FRB/FKBP12 regulation), and other novel CAR structures.

Further, the sequence of the hinge region in the CAR1 can be derived from: IgG, CD8, CD7, or CD4; the transmembrane domain in the CAR structure can be derived from: CD8, CD28, CD3ε, CD4, CD16, CD137, CD80 or CD86; the intracellular signaling domain in the CAR structure can be derived from: CD3, CD137, CD28, CD27, OX40, ICOS, GITR, CD2, CD40, PD-1, PD1L, B7-H3, lymphocyte function-associated antigen-1 (LFA-1), ICAM- 1. CD7, NKG2C, CD83, CD86 or CD 127.

Further, the ScFv recognizes any one of CD19, CD123, MOv-y, PSMA, IL13Rα2, EGFRvIII, EGFR, EPCAM, GD2, MUC1, HER2, GPC3, CEA, Meso, CD133, NKG2D, CD138, LeY, k-Light, CD33, ROR1, BCMA, CD30, CD20, CD22, PSCA, CLL-1, CD70, or CD47.

Further, the ScFv recognizes CD47, CEA, PSCA, CD19 or BCMA.

In certain embodiments, the CAR structure has a truncated EGFRt regulatory tag; in certain embodiments, the PSCA-targeting CAR structure is a universal CAR structure; in certain embodiments, the PSCA-targeting CAR structure carry suicide genes, such as iCasp9.

In certain embodiments, the CAR structure comprises one or more components of a natural killer cell receptor (NKR), thereby forming a NKR-CAR. The NKR component can be a transmembrane domain, hinge domain or cytoplasmic domain from any of the following natural killer cell receptors: killer cell immunoglobulin-like receptors (KIRs) (e.g., KIR2DL1, KIR2DL2/L3, KIR2DL4, KIR2DL5A, KIR2DL5B, KIR2DS1, KIR2DS2, KIR2DS3, KIR2DS4, DIR2DS5, KIR3DL1/S1, KIR3DL2, KIR3DL3, KIR2DP1, and KIR3DP1); natural cytotoxicity receptors (NCRs) (e.g., NKp30, NKp44, NKp46); signaling lymphocytic activation molecule (SLAM) family of immune cell receptors (e.g., CD48, CD229, 2B4, CD84, NTB-A, CRA, BLAME, and CD2F-10); Fc receptors (FcR) (e.g., CD16, and CD64); and Ly49 receptor (e.g., LY49A, LY49C). The NKR-CAR molecule can interact with an adaptor molecule or an intracellular signaling domain (e.g., DAP12).

As a preferred embodiment 1, the CAR structure is composed of the aforementioned SIRPγ fusion protein and the CAR1, wherein, the CAR1 comprises: the amino acid sequence of the hinge shown in SEQ ID NO: 24 or a functional variant thereof, the amino acid sequence of TM shown in SEQ ID NO: 7 or SEQ ID NO: 8, the amino acid sequence of CD3ζ shown in SEQ ID NO: 11 or a functional variant thereof; the fusion protein structure comprises: amino acid sequence of the extracellular domain of SIRPγ shown in SEQ ID NO: 1 or a functional variant thereof; the amino acid sequence of the transmembrane domain derived from human CD28 as shown in SEQ ID NO: 7; the amino acid sequence of the intracellular signaling domain derived from human CD28 as shown in SEQ ID NO: 9.

Further, the amino acid sequence of the ScFv is shown in SEQ ID NO: 25 or is a functional variant thereof.

Specifically, in this preferred solution, the gene-modified T lymphocytes expressing the novel immunosuppression-resistant CARs: ScFv-hinge-TM-CD3ζ-self-cleaving peptide-SIRPy-28TM-28, ScFv-hinge-TM-CD3ζ-self-cleaving peptide-SIRPy- 8TM-BB, ScFv-hinge-TM-4-1BB-CD3ζ-self-cleaving peptide-SIRPy-28TM-28, ScFv-hinge-TM-4-1BB-CD3ζ-self-cleaving peptide-SIRPy-8TM-BB and ScFv-hinge-TM-CD3ζ-self-cleaving peptide-PD-1-28TM-28 and ScFv-hinge-TM-CD3ζ-self-cleaving peptide-PD-1-8TM-BB are designed to verify the role of novel immunosuppression-resistant CARs.

Further, the method for preparing the CAR structure is: to co-express the CAR structure not comprising the SIRPγ fusion protein and the SIRPγ fusion protein in a vector when transfecting immune cells; or to express the CAR structure not comprising the SIRPγ fusion protein and the SIRPγ fusion protein separately in two vectors when transfecting immune cells.

As a preferred embodiment 2, the CAR structure is composed of the aforementioned SIRPγ fusion protein and the CAR1, wherein the CAR1 comprises an anti-CEA single chain antibody, a CD8 hinge region, a CD8 transmembrane domain, a CD137-CD3ξ dual-stimulus signal.

Preferably, the CAR structure is a CEA ScFv-CD8 hinge region-CD8 transmembrane domain-CD137-CD3ξ structure, the amino acid sequence of which comprises the sequence shown in SEQ ID NO:26. Wherein, the nucleic acid sequence encoding the CEA single chain antibody is shown in SEQ ID NO: 36; the nucleic acid sequence encoding the CD8 hinge region-CD8 transmembrane domain-CD137-CD3ξ structure is shown in SEQ ID NO: 37.

Further, the nucleic acid sequence encoding the CAR structure comprising a hypoxia inducible promoter comprises the sequence shown in SEQ ID NO:31.

As a preferred embodiment 3, the CAR structure is composed of the aforementioned SIRPγ fusion protein and the CAR1, wherein the CAR1 comprises a CD19 single chain antibody, a CD8 hinge region, a CD8 transmembrane domain, and a CD137-CD3ξ dual-stimulus signal;

Preferably, the amino acid sequence of the CAR structure is shown in SEQ ID NO: 27 or is a functional variant thereof.

Further, the nucleic acid sequence encoding the CAR structure comprises the sequence as shown in SEQ ID NO:32 or SEQ ID NO:33.

Further, the method for preparing the CAR structure is: to co-express the CAR structure not comprising the SIRPγ fusion protein and the SIRPγ fusion protein in a vector when transfecting immune cells; or to express the CAR structure not comprising the SIRPγ fusion protein and the SIRPγ fusion protein separately in two vectors when transfecting immune cells.

As a preferred embodiment 4, the CAR structure is composed of the aforementioned SIRPγ fusion protein and the CAR1, wherein, the CAR structure comprises a PSCA single-chain antibody, a hinge region, a CD28 transmembrane domain, a CD28-CD137-CD3ξ tristimulus signal; and the hinge region is G4H or 7H.

Preferably, the amino acid sequence of the CAR structure is shown in SEQ ID NO: 28 or is a functional variant thereof; or shown in SEQ ID NO: 29 or is a functional variant thereof. The amino acid sequence shown in SEQ ID NO: 28 or a functional variant thereof is a CAR structure with a hinge region of G4H; the amino acid sequence shown in SEQ ID NO: 29 or a functional variant thereof is a CAR structure with a hinge region of 7H.

Further, the nucleic acid sequence encoding the CAR structure comprises the sequence shown in SEQ ID NO: 34 or SEQ ID NO: 35; wherein SEQ ID NO: 35 comprises a hypoxia inducible promoter.

Further, the method for preparing the CAR structure is: to co-express the CAR structure not comprising the SIRPγ fusion protein and the SIRPγ fusion protein in a vector when transfecting immune cells; or to express the CAR structure not comprising the SIRPγ fusion protein and the SIRPγ fusion protein separately in two vectors when transfecting immune cells.

As a preferred embodiment 5, the CAR structure is composed of the aforementioned SIRPγ fusion protein and any one of the CAR1 in the preferred embodiment 1 to the preferred embodiment 4, the CAR structure also comprises a hypoxia inducible promoter, and the nucleic acid sequence of the hypoxia inducible promoter comprises the sequence shown in SEQ ID NO:30. The CD19-targeting CAR structure comprising a hypoxia inducible promoter can effectively remove tumors *in vivo* in a hypoxic microenvironment, and promoters induced by hypoxia microenvironment can enhance the expression of target genes, proteins and other factors in a hypoxic environment, and improve the drug efficacy on tumors and the efficacy and safety of CAR-T treatment, and not only can be effectively expressed in T lymphocytes, but also can enhance the expression of CAR molecules in hypoxic environment, and make CAR-T cells have high IFN-gamma secretion ability, improve the killing of CAR-T cells against tumor target cells, and can be used for targeted therapy of tumors.

Further, the hypoxia inducible promoter is formed by linking a Hifla regulatory element and a mini-promoter; the mini-promoter is selected from any one of a cytomegalovirus promoter, a promoter of HSV thymidine kinase, a promoter of simian virus 40, an adenovirus late promoter and a synthetic promoter.

Further, the method for preparing the structure of the CAR is: to co-express the CAR structure not comprising the SIRPγ fusion protein and the SIRPγ fusion protein in a vector when transfecting immune cells; or to express the CAR structure not comprising the SIRPγ fusion protein and the SIRPγ fusion protein separately in two vectors when transfecting immune cells.

The purpose of the present application is to further provide an expression vector comprising any of the aforementioned novel tumor immunosuppression-resistant CAR (including the embodiments in preferred embodiment 1 to preferred embodiment 5 and non-preferred embodiments), and immune cells comprising the expression vector.

Further, the expression vector is any one of a lentivirus expression vector, a retrovirus expression vector, an adenovirus expression vector, an adeno-associated virus expression vector, a DNA vector, an RNA vector, and a plasmid.

Further, the immune cells are T cells, T cell precursors or NK cells.

Further, the method for preparing the immune cells is: to co-express the CAR structure not comprising the SIRPγ fusion protein and the SIRPγ fusion protein in a vector when transfecting immune cells; or to express the CAR structure not comprising the SIRPγ fusion protein and the SIRPγ fusion protein separately in two vectors when transfecting immune cells.

In certain embodiments, the lentiviral vector is selected from the group consisting essentially of: human immunodeficiency virus 1 (HIV-1), human immunodeficiency virus 2 (HIV-2), Visna-maedi virus (VMV), goat arthritis-encephalitis virus (CAEV), equine infectious anemia virus (EIAV), feline immunodeficiency virus (FIV), bovine immunodeficiency virus (BIV) and simian immunodeficiency virus (SIV).

In certain embodiments, the vector comprises a left (5') retroviral LTR, a Psi(Ψ) packaging signal, a central polypurine tract/DNA flap (cPPT/FLAP), a retroviral export element, the promoter operably linked to the polynucleotides encoding the CARs included herein and a right (3') retroviral LTR.

In certain embodiments, the CAR comprises a hepatitis B virus post-transcriptional regulatory element (HPRE) or a woodchuck post-transcriptional regulatory element (WPRE) and an optimized woodchuck post-transcriptional regulatory element (oPRE).

In certain embodiments, the promoter in the 5' LTR is replaced with a heterologous promoter.

In certain embodiments, the heterologous promoter is a cytomegalovirus (CMV) promoter, a Rous Sarcoma Virus (RSV) promoter, or a simian virus 40 (SV40) promoter.

In certain embodiments, the 5' LTR or 3' LTR is a lentiviral LTR.

In certain embodiments, the 3' LTR is a self-inactivating (SIN) LTR.

In certain embodiments, the nucleic acid sequence of the CAR structure comprises an optimized Kozark sequence.

In certain embodiments, the promoter operably linked to a polynucleotide encoding a CAR included herein is a promoter selected from the group consisting of: cytomegalovirus immediate early gene promoter (CMV), elongation factor 1 alpha promoter (EF1-α), phosphoglycerate kinase-1 promoter (PGK), ubiquitin-C promoter (UBQ-C), cytomegalovirus enhancer/chicken β-actin promoter (CAG), multiple tumor virus enhancer/herpes simplex thymidine kinase gene promoter (MC1), β-actin promoter (β-ACT), simian virus 40 promoter (SV40) and myeloproliferative sarcoma virus enhancer, negative control region deleted, dl587rev primer-binding site substituted (MND) promoter.

In certain embodiments, the vector comprising a CAR comprises a secreted anti-PD-1 ScFv; in certain embodiments, the vector comprising a CAR comprises a PD-1 conjugated transduction peptide (e.g., PD-1-CD28-CD137-CD3 signaling structure); in certain embodiments, the vector comprising a CAR comprises a combination of multiple CARs, such as a combination of two CARs targeting different recognition sites of different antigens, or a combination of two CARs targeting different recognition sites of a same antigen.

The purpose of the present application is to further provide a pharmaceutical composition and use thereof.

For achieving the above object, the present application adopts the following embodiment:
The pharmaceutical composition comprises the fusion protein or an expression vector and immune cells comprising the fusion protein or the novel tumor immunosuppression-resistant CAR or the immune cells comprising the novel tumor immunosuppression-resistant CAR.

In certain embodiments, the fusion protein can be selected from SIRPα or a partial domain of SIRPα, and the structure of the fusion protein can be SIRPα-28TM-28, SIRPα-8TM-137, SIRPα-8TM-OX40, SIRPα-8TM-ICOS, etc. In some embodiments, the immune cells expressing the above-mentioned SIRPα fusion protein can be used alone, or the above-mentioned SIRPα fusion protein and the CAR molecule can be co-expressed in the same immune cell; or the immune cells expressing the SIRPα fusion protein and the CAR molecule respectively can be mixed in a certain proportion.

In certain embodiments, the active agent and/or treatment may be surgery, chemotherapy, radiation, immunosuppressive agents (e.g. cyclosporin, azathioprine, methotrexate, mycophenolate and FK506), antibodies or other immunoablative agents (e.g. CAMPATH, anti-CD3 antibody) or other antibody therapy, cytoxan, fludarabine, cyclosporin, FK506, rapamycin, mycophenolic acid, steroids, FR901228, cytokines and radiation.

In certain embodiments, the cells may express other active agents, e.g., active agents that enhance the activity of CAR-expressing cells. The active agent may be an active agent that blocks inhibitory molecules. Inhibitory molecules such as PD1 can in some embodiments reduce the ability of CAR-expressing cells to impose an immune effector response. Inhibitory molecules comprise PD1, PD-L1, CTLA4, TIM3, LAG3, VISTA, BTLA, TIGIT, LAIR1, CD160, 2B4, CEACAM (CEACAM-1, CEACAM-3, CEACAM-5), LAG3, VISTA, BTLA, TIG, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, TGFR (TGFRβ) and TGFRβ. The extracellular domain of the inhibitory molecule can be fused to a transmembrane domain and an intracellular signaling domain, such as PD1 CAR.

Further, the use of any one of the aforementioned fusion proteins or any one of the aforementioned CAR structures or any one of the aforementioned nucleic acid sequences or any one of the aforementioned expression vectors or any one of the aforementioned immune cells in the preparation of tumor drugs.

Further, the tumor is a malignant tumor, including acute lymphoid leukemia, chronic lymphocytic leukemia, chronic myeloid leukemia, non-Hodgkin's lymphoma, Hodgkin's lymphoma, prostate cancer, colorectal cancer, breast cancer, ovarian cancer, cervical cancer, pancreatic cancer, lung cancer, kidney cancer, liver cancer, brain cancer or skin cancer.

In the present application, in the CAR structure, Z refers to the human CD3 intracellular signal CD3ζ, BB refers to the human 4-1BB intracellular signal, BBZ refers to the intracellular domain of 4-1BB ICD-CD3ζ, and 28Z refers to the intracellular domain of CD28ICD-CD3ζ.

In the present application, the "functional variant" generally refers to an amino acid sequence comprising substantially the same function (for example, it can have the properties of the chimeric antigen receptor) and at least 85% (for example, at least 85%, at least 90%, at least 91%, at least 92%, at least 93%, at least 94%, at least 95%, at least 96%, at least 97%, at least 98%, at least 99%, or at least 100%) sequence identity with it. In certain embodiments, the variant of the amino acid sequence has substantially the same function as the amino acid sequence.

In the present application, in the CAR structure, CEAZ refers to a CEA-targeting CAR structure with only CD3ζ in its intracellular structure, and CEA-28Z refers to a CEA-targeting CAR structure with a co-stimulatory signal derived from the intracellular domain of CD28 and CD3ζ in its intracellular structure, CEA-BBZ refers to a CEA-targeting CAR structure with a co-stimulatory signal derived from the intracellular domain of 4-1BB and CD3ζ in its intracellular structure, BCMA-BBZ refers to a BCMA-targeting CAR structure with a co-stimulatory signal derived from the intracellular domain of 4-1BB and CD3ζ in its intracellular structure; SIRPy-28TM-28 refers to a SIRPγ fusion peptide with only a signal derived from the intracellular domain of CD28 in its intracellular structure, and abbreviated as SIRPy-28; SIRPγ-8TM-BB refers to a SIRPγ fusion peptide with only a signal derived from the intracellular domain of 4-1BB in its intracellular structure, and abbreviated as SIRPγ-BB.

The beneficial effects of the present application are:
The fusion protein for reversing the tumor microenvironment provided by the present application can reverse the tumor microenvironment and target, targeted-killing CD47-positive tumor cells; the novel tumor immunosuppression-resistant CAR comprising the fusion protein and the immune cells break down the influence of the inhibitory signal on CAR-T function in the tumor tissue, realizing the effectiveness of CAR-T treatment and ensuring certain safety.

### BRIEF DESCRIPTION OF THE DRAWINGS

Figure 1 is a structural diagram of an immunosuppression-breaking fusion protein.
Figure 2 is a schematic diagram of the structure of an immunosuppression-resistant CAR.
Figure 3 is the picture of the verification of CAR construction plasmids.
Figure 4 shows the result of target cell constructions.
Figure 5a shows the CAR positive rate of the novel immunosuppression-resistant CAR-T.
Figure 5b shows the expression intensity of the novel immunosuppression-resistant CAR-T.
Figure 6a shows the *in vitro* function verification of the novel immunosuppression-resistant CAR-T.
Figure 6b shows the *in vitro* killing rate of the novel immunosuppression-resistant CAR-T.
Figure 7 shows the validation of the novel immunosuppression-resistant CAR-T to disrupt the tumor suppressive environment.
Figure 8a shows the triple positive expression of CEA, PD-L1 and CD47 in NCG mice.
Figure 8b plots the tumor volume growth curve for the tumor volume measurement data of NCG mice.
Figure 9 shows the *in vivo* effectiveness of the second-generation immunosuppressive CAR.
Figure 10 shows the average fluorescence intensity of each experimental group targeting CEA.
Figure 11 shows the positive rate of each experimental group targeting CEA.
Figure 12 is the *in vitro* expansion fold curve of CAR-T in each experimental group targeting CEA.
Figure 13 shows the killing efficiency of CAR-T in each experimental group targeting CEA on target cells DLDL1-CEA.
Figure 14 shows the secretion of IFN-γ in DLD1-CEA and DLD1-CEA (CD47-) cells in each experimental group targeting CEA.
Figure 15 shows the secretion of IL-2 in DLD1-CEA and DLD1-CEA (CD47-) cells in each experimental group targeting CEA.
Figure 16 shows the secretion of TNF-α in DLD1-CEA and DLD1-CEA (CD47-) cells in each experimental group targeting CEA.
Figure 17 is an image of the tumor growth in *in vivo* hypoxia verification model mice in the control and screening groups targeting CEA.
Figure 18 shows the bioluminescence in *in vivo* hypoxia verification model mice in the control and screening groups targeting CEA
Figure 19 shows the detection of the positive rate of CAR-T in each experimental group targeting CD19 by flow cytometry.
Figure 20 shows the cell killing of each experimental group targeting CD19.
Figure 21 shows the secretion of IFN-γ factor in each experimental group targeting CD19.
Figure 22 shows the *in vivo* bioluminescence of mice in each experimental group targeting CD19.
Figure 23 shows tumor growth of mice in each experimental group targeting CD19.
Figure 24 shows the secretion of IFN-γ factor by PSCA-28BBZ-G4H-28TM-P2A-SIRPγ-28.
Figure 25 shows the secretion of IFN-γ factor by PSCA-28BBZ-7H-28TM-P2A-SIRPγ-28.
Figure 26 shows the secretion of IFN-γ factor by PSCA-28BBZ-G4H-28TM+SIRPγ-28.
Figure 27 shows the secretion of IFN-γ factor by PSCA-28BBZ-7H-28TM+SIRPγ-28.
Figure 28 shows the secretion of IFN-γ factor by PSCA-28BBZ-G4H-28TM and PSCA-28BBZ-G4H-28TM+SIRPγ-28.
Figure 29 shows the secretion of IFN-γ factor by PSCA-28BBZ-7H-28TM and PSCA-28BBZ-7H-28TM+SIRPγ-28.
Figure 30 shows the cell killing of PSCA-28BBZ-G4H-28TM-P2A-SIRPγ-28 in positive/negative cells, respectively.
Figure 31 shows the cell killing of PSCA-28BBZ-7H-28TM-P2A-SIRPγ-28 in positive/negative cells, respectively.
Figure 32 shows the cell killing of PSCA-28BBZ-G4H-28TM+SIRPγ-28 in positive/negative cells, respectively.
Figure 33 shows the cell killing of PSCA-28BBZ-7H-28TM+SIRPγ-28 in positive/negative cells, respectively.
Figure 34 shows the cell killing of PSCA-28BBZ-G4H-28TM and PSCA-28BBZ-G4H-28TM+SIRPγ-28.
Figure 35 shows the cell killing of PSCA-28BBZ-7H-28TM and PSCA-28BBZ-7H-28TM+SIRPγ-28.

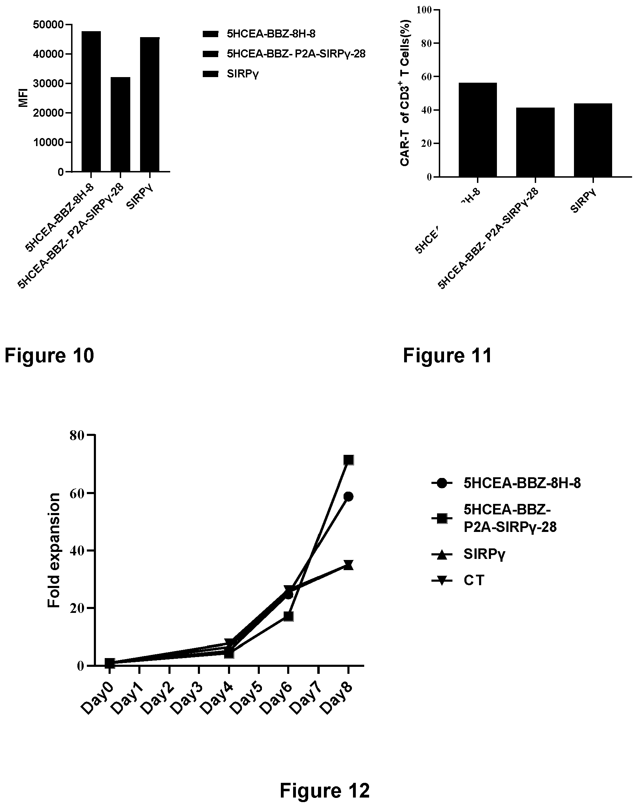

In Figures 10-18, 5HCEA-BBZ refers to a CEA-targeting CAR structure with a Hypoxia inducible promoter and a co-stimulatory signal derived from the 4-1BB intracellular domain and CD3ζ in its intracellular structure; CEA-BBZ refers to a CEA-targeting CAR structure with a co-stimulatory signal derived from the intracellular domain of 4-1BB and CD3ζ in its intracellular structure, CEA-28Z refers to a CEA-targeting CAR structure with a co-stimulatory signal derived from the intracellular domain of CD28 and CD3ζ in its intracellular structure, SIRPy-28TM-28 refers to a fusion peptide with only a signal derived from the intracellular domain of CD28 in its intracellular structure, abbreviated as SIRPy-28.

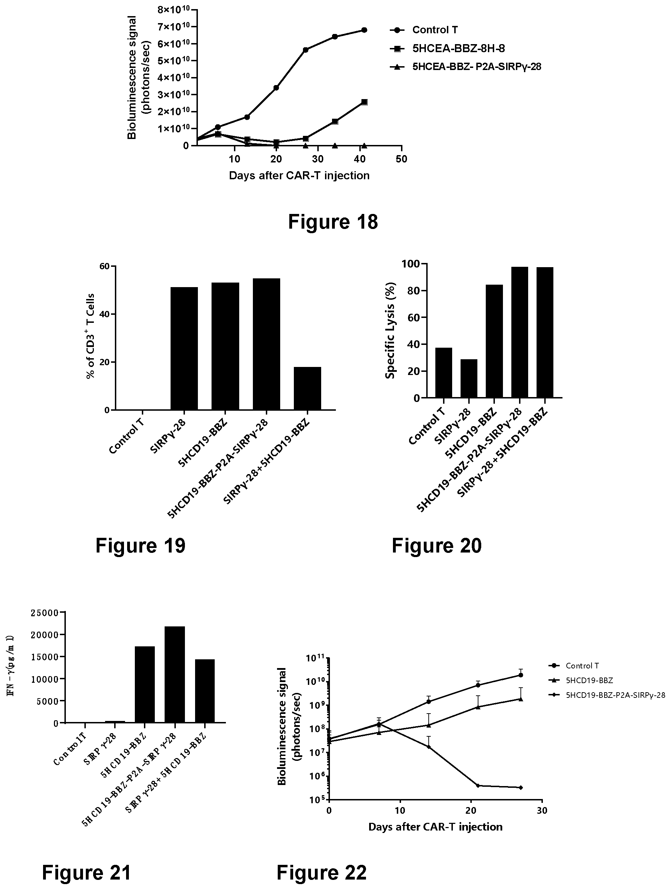

In Figures 19-23, 5HCD19-BBZ refers to a CD19-targeting CAR structure with a Hypoxia inducible promoter, a co-stimulatory signal derived from the intracellular domain of 4-1BB (abbreviated as BB) and CD3ζ in its intracellular structure; SIRPy-28TM-28 (SIRPy-28) refers to a fusion peptide with an only intracellular signal from CD28; SHCD19-BBZ-SIRPγ-28 refers to a CAR with the following structure: SHCD19-8H-8TM-CD137-CD3ζ; SIRPγ-28-5HCD19-BBZ refers to a CAR-T obtained by transfecting immune cells with the CAR comprising a hypoxia inducible promoter and the fusion protein expressed by two vectors respectively.

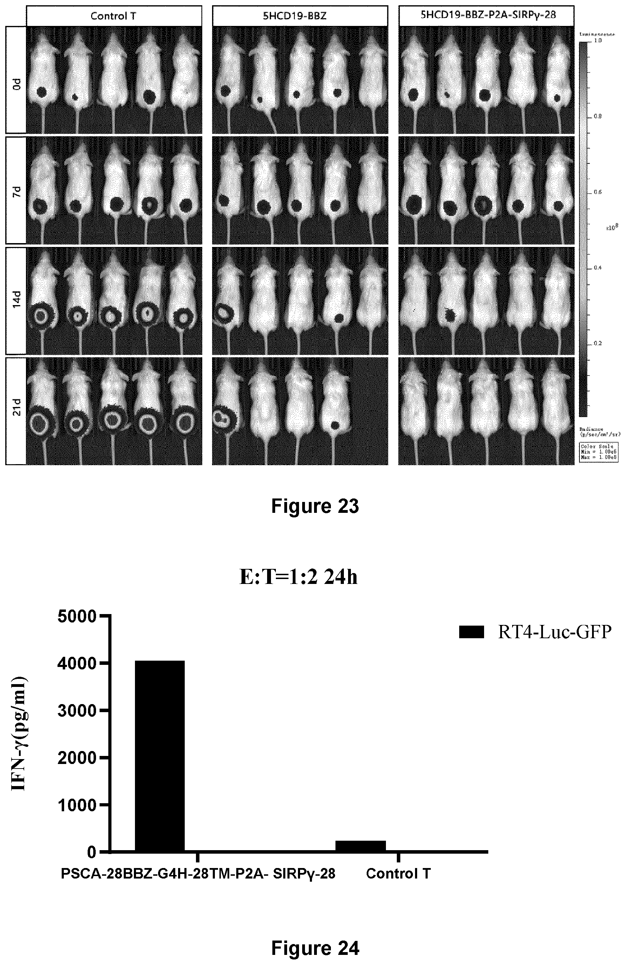

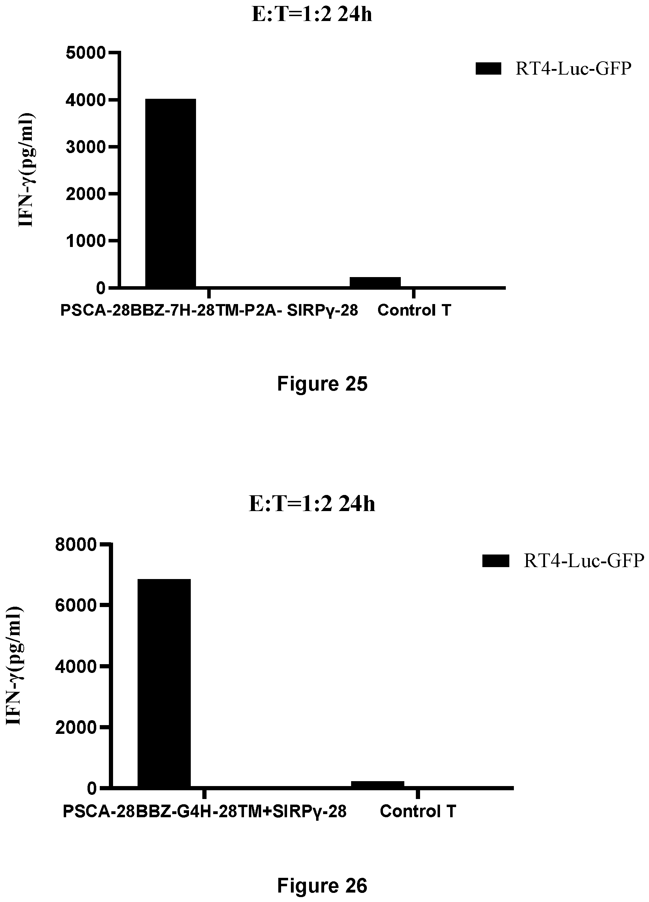

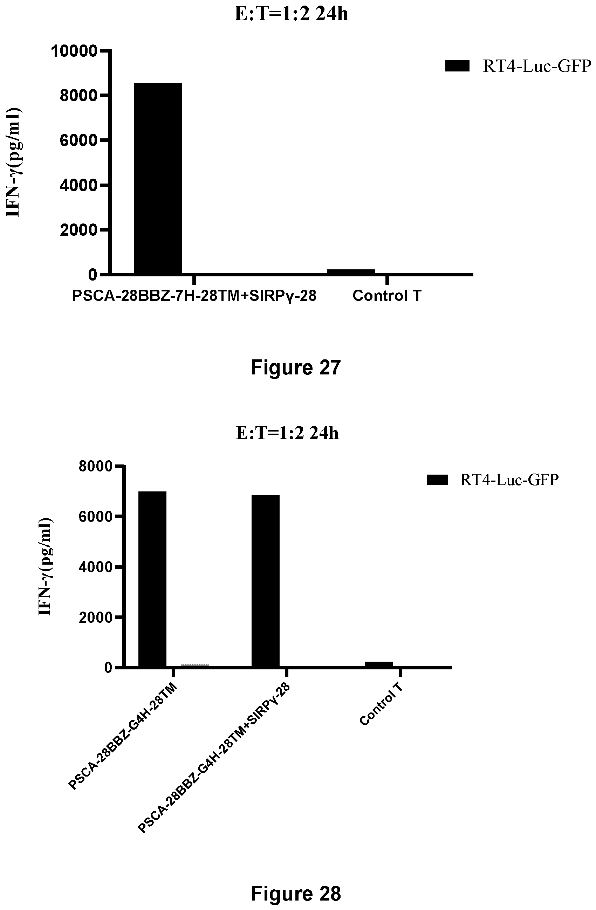

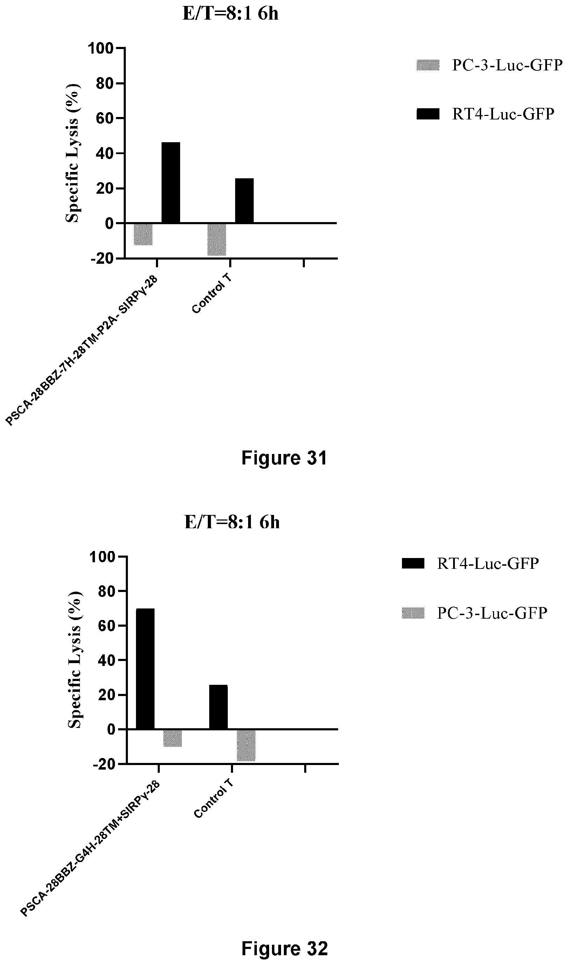

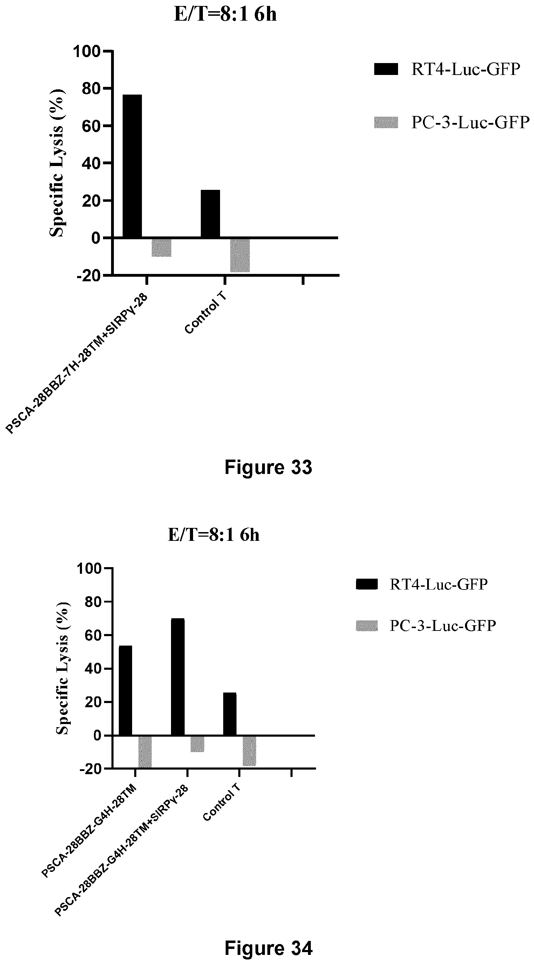

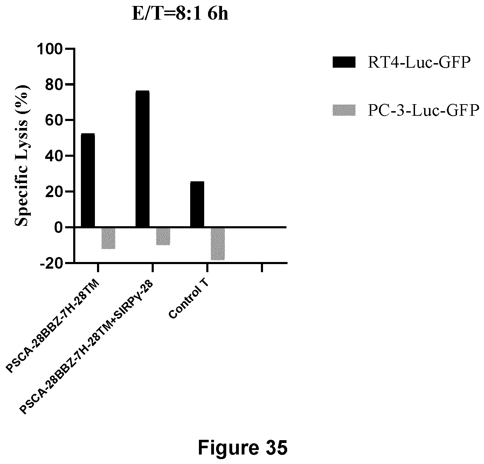

In Figures 24-35, RT4-Luc-GFP is a positive cell, and PC-3-Luc-GFP is a negative cell; SIRPy-28TM-28 (SIRPy-28) refers to a fusion peptide with only the signal derived from the intracellular domain of CD28 in its intracellular structure; PSCA-28BBZ-G4H-28TM+SIRPγ-28 or PSCA-28BBZ-7H-28TM+SIRPγ-28 refers to the products obtained by transfecting immune cells with two vectors expressing PSCA-28BBZ-G4H-28TM or PSCA-28BBZ-7H-28TM with the fusion protein SIRPy-28 separately. PSCA-28BBZ-G4H-28TM-P2A-SIRPγ-28 or PSCA-28BBZ-7H-28TM-P2A-SIR.Pγ-28 refers to the products obtained by transfecting immune cells with a vector co-expressing PSCA-28BBZ-G4H-28TM or PSCA-28BBZ-7H-28TM with the fusion protein SIRPy-28.

### DETAIL DESCRIPTION OF THE INVENTION

Hereinafter, preferred examples of the present application will be described in detail with reference to the drawings. In the preferred examples, the experimental methods that do not specify specific conditions are usually in accordance with conventional conditions, such as the conditions described in the Molecular Cloning: A Laboratory Manual (third edition, J. Sambrook et al.), or as recommended by the manufacturer. The listed examples are used to better illustrate the content of the present application, but the content of the present application is not limited to the listed examples. Therefore, those skilled in the art make non-essential improvements and adjustments to the embodiments according to the above-mentioned contents of the application, resulting in which still belong to the protection scope of the present application.

In the examples of the present application, the sequences involving vector structure elements are shown in Table 1 below.

**Table 1 Sequences of the vector structure elements.**

| CAR structure element | Nucleotide sequence | Amino acid sequence |
|---|---|---|
| SIRPγ | SEQ ID NO:12 | SEQ ID NO: 1 |
| hinge | | SEQ ID NO:24 |
| CD28TM | SEQ ID NO: 18 | SEQ ID NO:7 |
| CD8TM | SEQ ID NO: 19 | SEQ ID NO:8 |
| CD28 ICD | SEQ ID NO:20 | SEQ ID NO:9 |
| 4-1BB ICD | SEQ ID NO:21 | SEQ ID NO:10 |
| CD3ζ | SEQ ID NO:22 | SEQ ID NO:11 |
| CEA ScFv | | SEQ ID NO:25 |
| CEAZ | SEQ ID NO:15 | SEQ ID NO:4 |
| CEA-BB-CD3ζ | | SEQ ID NO:23 |
| SIRPγ-28TM-28 | SEQ ID NO:13 | SEQ ID NO:2 |
| SIRPγ-8TM-BB | SEQ ID NO:14 | SEQ ID NO:3 |
| CEAZ-P2A-SIRPγ-28 | SEQ ID NO:16 | SEQ ID NO:5 |
| CEAZ-P2A-SIRPγ-BB | SEQ ID NO:17 | SEQ ID NO:6 |

In the examples of the present application (in Table 1): Z refers to CD3ζ, CEAZ refers to the CAR structure (ScFv(CEA)-hinge-TM-CD3ζ) with only the CD3ζ in the intracellular structure, and CEA-28Z refers to a CEA-targeting CAR structure (ScFv(CEA)-hinge-TM-CD28-CD3ζ) with a co-stimulatory signal derived from the intracellular domain of CD28 and CD3ζ in its intracellular structure, CEA-BBZ refers to a CEA-targeting CAR structure (ScFv(CEA)-hinge-TM-4-1BB-CD3ζ) with a co-stimulatory signal derived from the intracellular domain of 4-1BB and CD3ζ in its intracellular structure, BCMA-BBZ refers to a BCMA-targeting CAR structure with a co-stimulatory signal derived from the intracellular domain of 4-1BB and CD3ζ in its intracellular structure; SIRPy-28TM-28 refers to a fusion peptide with only a signal derived from the intracellular domain of the CD28 in its intracellular structure, abbreviated as SIRPy-28; SIRPy-8TM-BB refers to a fusion peptide with only a signal derived from the intracellular domain of 4-1BB in its intracellular structure, abbreviated as SIRPγ-BB.

In the examples of the present application, 5HCD19-BBZ refers to a CD19-targeting CAR structure with a Hypoxia inducible promoter, a co-stimulatory signal derived from the intracellular domain of the 4-1BB (abbreviated as BB) and the CD3ζ in its intracellular structure; SIRPy-28TM-28 (abbreviated as SIRPy-28) refers to a fusion peptide with only a signal derived from the intracellular domain of the CD28 in its intracellular structure; SIRPy-28+5HCD19-BBZ refers to a CAR-T obtained by transfecting immune cells with two vectors expressing the CAR comprising a Hypoxia inducible promoter and the fusion proteins separately. 5HCD19-BBZ-P2A-SIRPγ-28 refers to a CAR-T obtained by transfecting immune cells with a vector co-expressing the CAR comprising a hypoxia inducible promoter and the fusion protein.

In the examples of the present application, PSCA-28BBZ-G4H-28TM+SIRPγ-28 or PSCA-28BBZ-7H-28TM+SIRPγ-28 refers to the products obtained by transfecting immune cells with two vectors expressing PSCA-28BBZ-G4H-28TM or PSCA-28BBZ-7H-28TM with the fusion protein SIRPy-28 separately. PSCA-28BBZ-G4H-28TM-P2A-SIRPγ-28 or PSCA-28BBZ-7H-28TM-P2A-SIR.Pγ-28 refers to the products obtained by transfecting immune cells with a vector co-expressing PSCA-28BBZ-G4H-28TM or PSCA-28BBZ-7H-28TM with the fusion protein SIRPy-28.

In the examples of the present application, the method for packaging lentivirus by calcium phosphate method is specifically: 293T cells are cultured to a better state with DMEM medium comprising 10% FBS (w/v), the packaging plasmids (RRE: REV: 2G) and the expression plasmid are added to a 1.5 centrifuge tube according to a certain ratio, and CaCl2 and 2×HBS are added. After being well mixed, cells are added to the treated 293T cell culture medium after being left to stand at room temperature. After 3-5 hours, the medium is changed again to 10mL DMEM medium with 10% FBS, and the cell supernatant is collected after 48 h or 72 h, and the virus is purified.

In the examples of the present application, the antibody is: Protein-L-PE. Protein-L can recognize the light chain of the antibody, and the light chain of the ScFv sequence in the CAR antigen recognition region can be recognized by Protein-L. So the Protein-L can be used to detect CAR positive rate and CAR expression intensity. SIRPy-28 is tagged with GFP, and its expression is determined by the GFP positive rate.

In the examples of the present application, the method for detecting the killing ability of different CAR-Ts on target cells is as follows: using the ACEA xCELLigence RTCA MP instrument, and the experimental steps are performed according to the instructions of the instrument. The principle of ACEA xCELLigence RTCA MP is using the resistance index data to record the tumor cells attached to the bottom of the well every 15 minutes, and the proliferation or death of the adherent target cells is determined according to the resistance index. The results are analyzed by using the resistance index, the formula is: CAR-T cell killing rate = baseline resistance index - real-time resistance index.

In the examples of the present application, IFN-γ is detected with BD IFN-γ kit, and the experimental steps are carried out according to the product instructions; IL-2 is detected with Invitrogen IL-2 kit, and the experimental steps are carried out according to the product instructions; TNF-α is detected with the Biolegend kit, and the experimental steps are carried out according to the product instructions.

In the examples of the present application, the method for verifying whether the construction of the hypoxia model has been achieved is as follows: an *in vitro* hypoxic cell model is constructed by infection of activated PBMC with recombinant plasmid virus, and after culturing for 12-18 hours, the medium is changed; then a hypoxic environment is induced by CoCl2, and, the expression of CAR is detected by detecting the light chain antibody on the CAR structure on day N of the culture.

In the examples of the present application, the mice used for *in vivo* verification are NOD. Cg-PrkdcscidII2rgtm1Sug/JicCrl is abbreviated as NOG mice, which are bred by Mamoru Ito of the Central institute of Experimental Animals (CIEA), Japan, and are the most common strain of CAR-T related tumorigenesis *in vivo* experiment in the world.

In the examples of the present application, the method for verifying with the hypoxia model *in vivo* is as follows: 6-8-week old female NOG mice are selected, after marking the ear numbers, on the back of the mouse, target cells are injected subcutaneously at an amount of 1×10^6 cells/mouse, the tumor volume of mice is measured on the 12th day after tumor formation.

### Part I

### Example 1 Plasmid construction

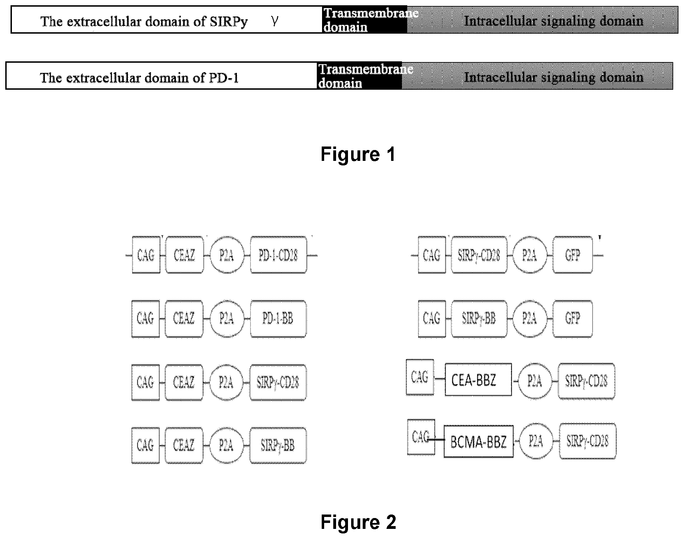

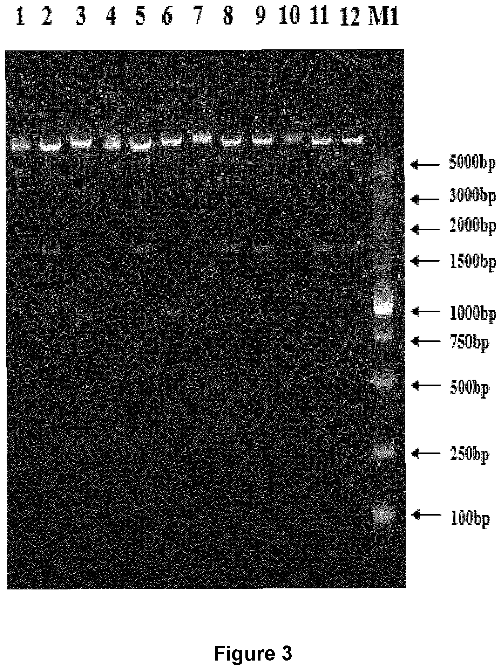

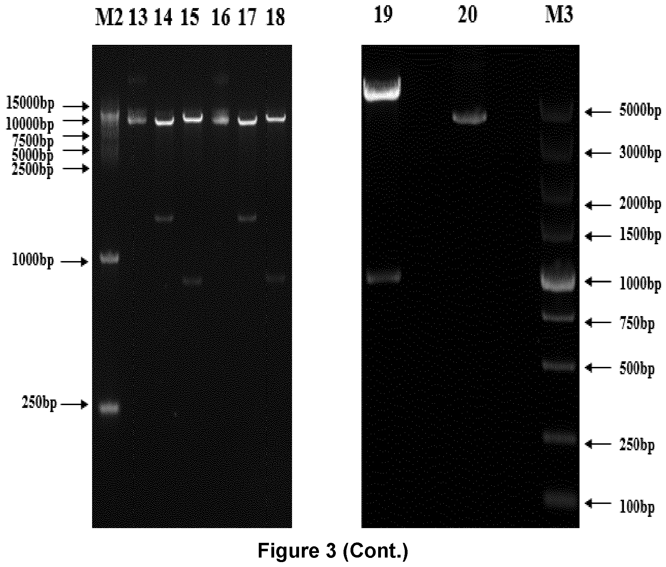

Based on the CAR pattern shown in Figure 1, using SIRPγ and CD47 full-length plasmids, pL-CAG-2AGFP, pL-CAG-PD1-CD28-2ACherry, and pL-CAG-PD1-BB-2ACherry as templates, the CAR structure shown in Figure 2 and the corresponding single-target CAR structure were constructed. The vectors: CEAZ-PD1-28, CEAZ-PD1-BB, CEAZ-SIRPγ-BB, CEAZ-SIRPγ-28, CEABBZ-P2A-SIRPγ-28, CEABBZ-P2A-SIRPy-BB, BCMA-BBZ-P2A- SIRPy-28 and single-target CARs: CEAZ, PD1-28, PD1-BB, SIRPγ-28TM-28, SIRPy-8TM-BB were obtained by construction. After verification with enzyme digestion and sequencing and alignment, the results were shown in Figure 3. The enzyme digestion identification of the recombinant plasmid: 1-3: pL-CAG-CEAZ-PD1-28 plasmid, the order is: original plasmid, CEAZ (1371bp), PD1-28 (783bp); 4-6: pL-CAG-CEAZ-PD1-BB plasmid, the order is: original plasmid, CEAZ (1371bp), PD1-BB (798bp); 7-9: pL-CAG-CEAZ- SIRPy-28 plasmid, the order is: original plasmid, CEAZ (1371bp), SIRPy-28 (1374bp); 10-12: pL-CAG-CEAZ-SIRPy-BB plasmid, the order is: original plasmid, CEAZ (1371bp), SIRPγ-BB (1368bp); M1: DL5000 DNA molecular weight standard; M2: DL15000 DNA molecular weight standard; 13-15: pL-CAG-SIRPy-28-2AGFP plasmid, the order is: original plasmid, SIRPy-28 (1292bp), 2AGFP (785bp) 16-18: pL-CAG-SIRPy-BB-2AGFP plasmid, the order is: original plasmid, SIRPγ-BB (1286bp), 2AGFP (785bp) 19-20: pL-CAG-CD47 plasmid, the order is: CD47 (974bp), the original plasmid; M3: DL5000 DNA molecular weight standard; the construction was successful.

### Example 2 Target cell construction

Viruses with CEA, PD-L1 and CD47 antigens were prepared by calcium phosphate method, and CHO cells were infected to construct CHO-CEA cells, CHO-CEA-PD-L1 cell lines and CHO-CEA-CD47 cell lines, respectively. Signal regulatory protein gamma (SIRPγ) was one of the ligands of CD47 and can bind to CD47, so CD47-positive target cells could be used to evaluate CEAZ-SIRPγ-BB, CEAZ-SIRPy-28, SIRPγ, SIRPy-28, and SIRPγ-BB.

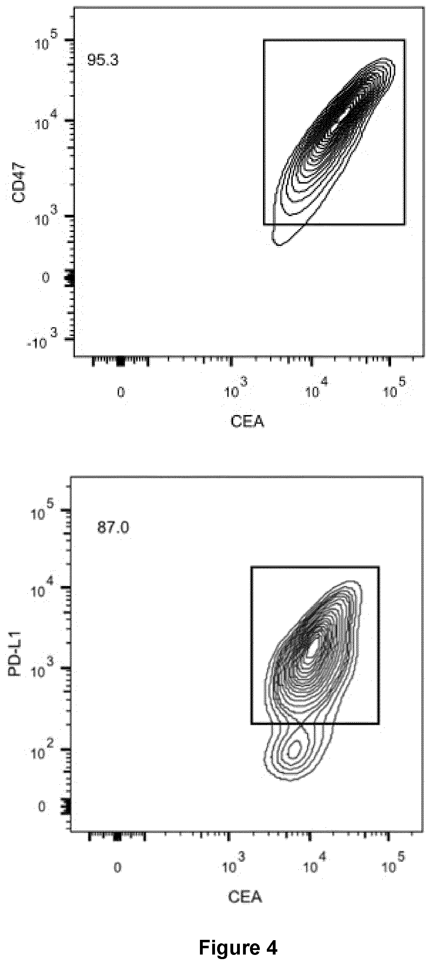

The positive rate of the three cell lines was detected after ten subcultures. The results were shown in Figure 4. The positive rate of CHO-CEA was 97.1%, the double-positive rate of CHO-CEA-CD47 was 97.6%, and the positive rate of CHO-CEA-PD-L1 was 87%, which meets the experimental requirements, indicating that the cell lines had been successfully constructed and could be used as target cells for subsequent CAR-T efficacy evaluation.

### Example 3 Preparation of lentivirus and infection of T lymphocytes

The lentivirus was packaged by the calcium phosphate method to obtain 5 virus particles with single-expressed CARs (CEAZ, PD1-28, PD1-BB, SIRPy-28, SIRPγ-BB) and 6 virus particles with novel immunosuppression-resistant CARs (CEAZ-PD1-28, CEAZ-PD1-BB, CEAZ-SIRPy-28, CEAZ-SIRPγ-BB, CEABBZ-SIRPγ-28, CEABBZ-SIRPγ-BB).

Gradient centrifugation was used to separate lymphocytes; after centrifugation, the second layer with white lymphocytes was taken, washed with physiological saline, and cultured by adding RPMI 1640 complete medium comprising 10% FBS to obtain human PBMC cells. The obtained PBMC cells were activated by anti-CD3 and CD28 monoclonal antibodies for 24 hours, the activated PBMCs were infected with a certain multiplicity of infection (MOI), and the positive rate of CAR-T was detected on the 12th day of virus infection. The detection method was flow cytometry, the antibody was: Protein-L-PE, Protein-L could recognize the light chain of the antibody, and the light chain of the ScFv sequence of the CAR antigen recognition region could be recognized by Protein-L, so Protein-L could be used to detect CAR positive rate and the density of CAR expression.

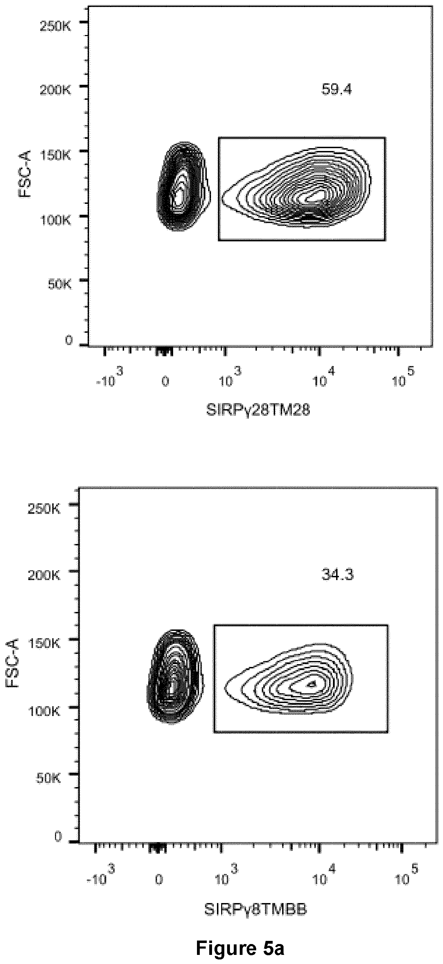

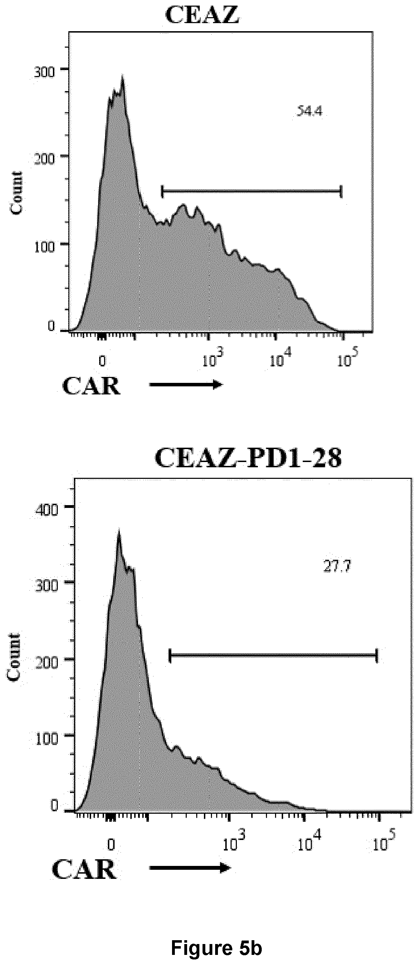

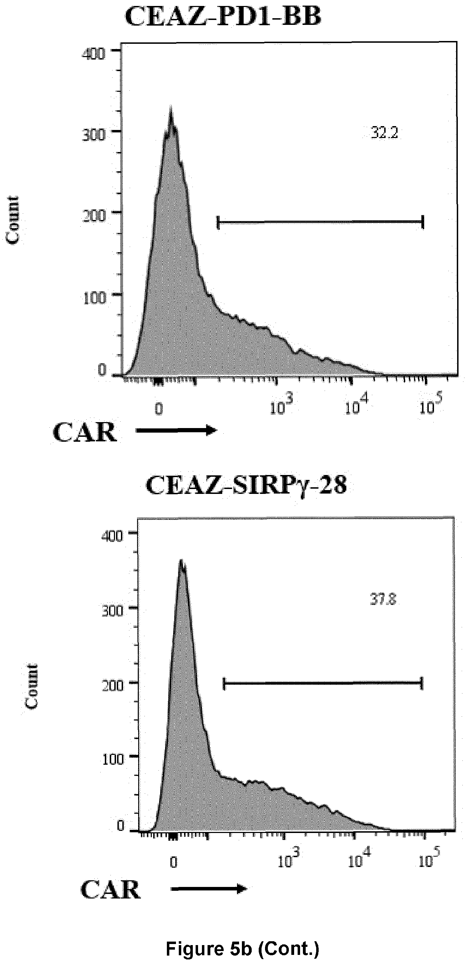

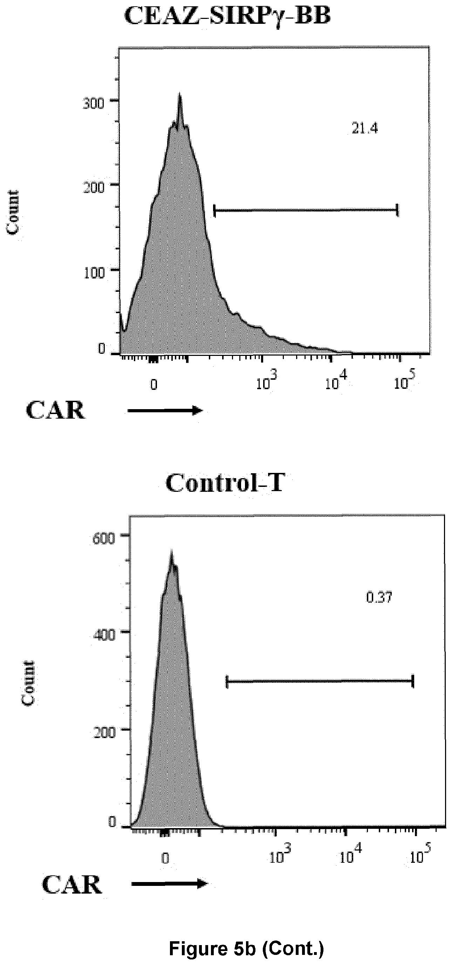

The results were shown in Figure 5a: the immunosuppression-disrupting fusion protein could be successfully expressed on the surface of T cells, as shown in Figure 5b, the novel immunosuppression-resistant CARs were successfully expressed.

### Example 4 In vitro function verification of novel immunosuppression-resistant CAR-T

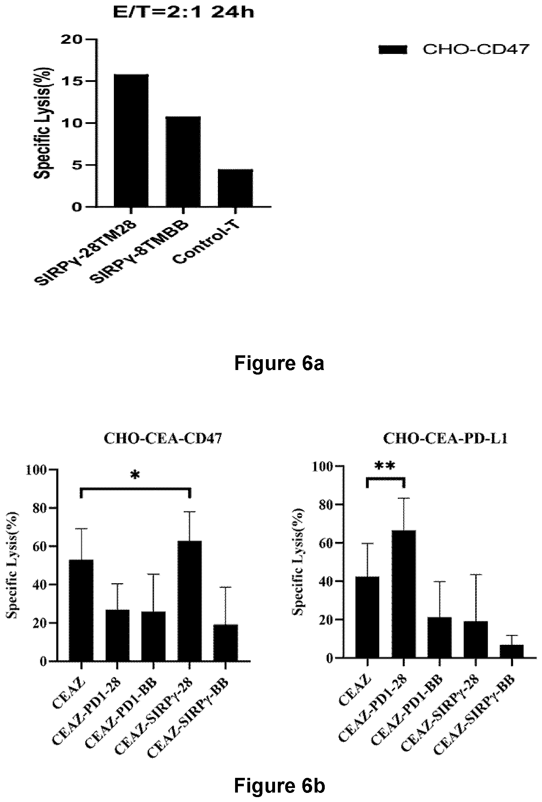

Control T cells that did not express the immunosuppression-disrupting fusion protein were used as a control to verify the functions of the immunosuppressive-disrupting fusion proteins SIRPy-28TM-28 and SIRPy-8TM-BB. The target cells were the CHO cell line expressing CD47. The results were shown in Figure 6a, SIRPy-28TM-28 and SIRPγ-8TM-BB fusion proteins had killing effects on CD47-positive target cells.

Taking CEAZ group as the positive control and Control-T group as the negative control, CEAZ-PD1-28, CEAZ-PD1-BB, CEAZ-SIRPy-28, CEAZ-SIRPγ-BB groups were set as experimental groups, and CHO-CEA-CD47 and CHO-CEA-PD-L1 were set as target cells to verify the *in vitro* effectiveness of the novel immunosuppression-resistant CAR-Ts. The results were shown in Figure 6b and Table 2 below, the cell killing efficiency of the CEAZ-PD1-28 and CEAZ-SIRPγ-28 groups were significantly higher than that of the CEAZ group, while the killing efficiency of the CEAZ-PD1-BB and CEAZ-SIRPγ-BB groups was not significantly enhanced compared with that of CEAZ group. In addition, in CEAZ-PD1-28 and CEAZ-PD1-BB groups, in the case of co-culturing with CHO-CEA-CD47, and in CEAZ-SIRPγ-28 and CEAZ-SIRPγ-BB groups, in the case of co-culturing with CHO-CEA-PD-L1, there was no significant difference in the killing efficiency compared with that of the CEAZ group, indicating that the novel immunosuppression-resistant CAR-Ts had specific killing, and CEAZ-PD1-28 and CEAZ-SIRPy-28 had significant killing effects on target cells.

**Table 2 In vitro killing efficiency of immunosuppression-resistant CAR-T**

| Structure | Target cell | |
|---|---|---|
| | CHO-CEA-CD47 (specific lysis%) | CHO-CEA-PDL1 (specific lysis%) |
| CEA-Z | 52.95458±16.246 | 42.37838±17.43639 |
| CEA-Z-PD1-28 | 26.8274±13.62647 | 66.58775±16.8083 |
| CEA-Z-PD1-BB | 25.88708±19.55145 | 21.18065±18.72001 |
| CEA-Z-SIRPγ-28 | 62.8963±15.15207 | 19.26225±24.32014 |
| CEA-Z-SIRPγ-BB | 19.08425±19.57656 | 6.92085±4.83563 |

Using K562-BCMA as the target cell and Control-T group as the negative control, the function of BCMA-BBZ-P2A-SIRPy-28 was verified, and the result showed BCMA-BBZ-P2A-SIRPy-28 could show good killing function.

Example 5 Validation of a new type of immunosuppression-resistant CAR-T to disrupt the tumor suppressive environment

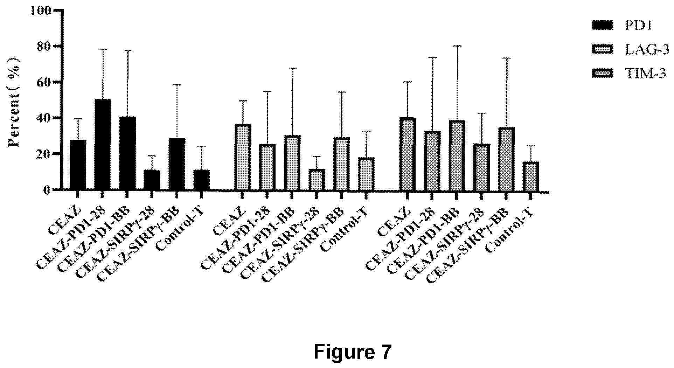

After CAR-T cells infiltrated tumor tissues, they were often affected by tumor immunosuppressive microenvironment, and highly expressed attenuating molecules PD1, LAG-3, and Tim-3 a, thereby the effective function of killing tumor cells was weakened, and the apoptosis of CAR-T cells themselves was increased. In order to verify whether the novel immunosuppression-resistant CAR-T could break the tumor suppressive microenvironment signal, in CEAZ, CEA-28Z, CEA-BBZ, CEAZ-PD1-28, CEAZ-PD1-BB, CEAZ-SIRPγ-28, CEAZ-SIRPγ-BB and Control-T groups, when CAR-T cells were cultured to Day 7, CAR-T cells and DLD-1-CEA-Luc-GFP cells were co-cultured in a 12-well cell culture plate, and CAR-T cells were collected after 48 hours , labeled with anti-human CD3, PL, PD1, LAG-3, Tim-3 flow cytometry antibody, and then detected by flow cytometry, and the detection results were analyzed to evaluate their effect ability. Since PD1 was exogenously expressed in the CEAZ-PD1-28 and CEAZ-PD1-BB groups, the PD1 positivity rates of these two groups were not used to evaluate the degree of CAR-T cell exhaustion. The results of exhausted molecular detection were shown in Figure 7 and Table 3 below: Under the condition of CD3-PL+, the expression levels of PD1, LAG-3 and Tim-3 in CEAZ-SIRPγ-28 group were lower than those in CEAZ group, indicating that the degree of exhaustion in CEAZ-SIRPy-28 after antigen stimulation was lower.

**Table 3. Validation of immunosuppression-resistant CAR-T against tumor suppressive environment**

| Structure | Expression value of exhausted molecules | | |
|---|---|---|---|
| | PD1 | LAG-3 | TIM-3 |
| CEAZ | 27.93333±11.55783 | 37.36667±12.72019 | 41.26667±19.85254 |
| CEAZ-PD1-28 | 50.56667±28.00042 | 26.02667±29.26886 | 34.11333±40.76119 |
| CEAZ-PD1-BB | 40.93333±36.89205 | 31.36±37.05815 | 39.86667±41.51293 |
| CEAZ-SIRPγ-28 | 11.27667±7.901559 | 12.28667±7.190447 | 27.06667±16.64041 |
| CEAZ-SIRPγ-BB | 29.49667±29.36888 | 30.2±25.03937 | 36.44±38.31466 |
| Control-T | 11.65667±13.05796 | 19.05±14.40113 | 17.20667±8.675606 |

Likewise, BCMA-BBZ-P2A-SIRPy-28 also showed lower expression of exhausted molecules after stimulation with BCMA antigen.

### Example 6 In vivo functional verification of novel immunosuppression-resistant CAR-T

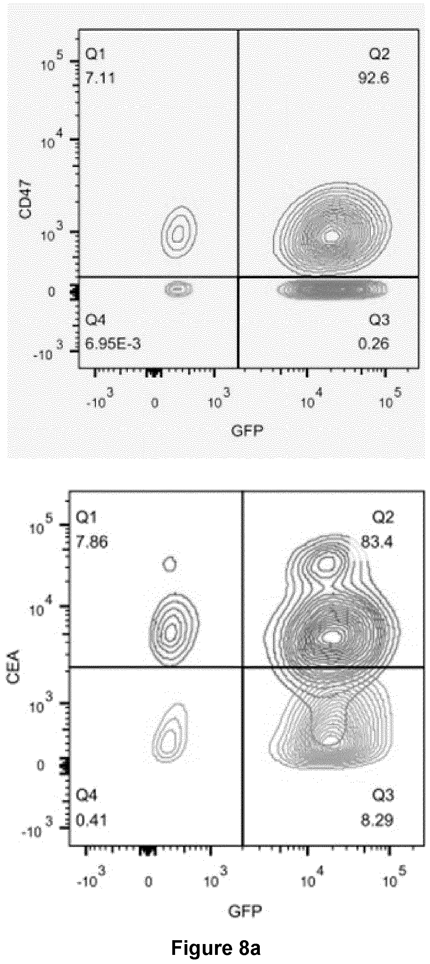

The mice used for *in vivo* validation were NCG mice. Thirty NCG mice were selected and injected with DLD-1-CEA-Luc-GFP (CEA, PD-L1, CD47 triple positive expression, as shown in Figure 8a) for tumor bearing. When the tumor grew to a measurable size as mung bean, the tumor size is measured. During the experiment, when the mice in the experimental group appeared any situation of: sluggish and dying, half- or whole-body paralysis, lost 20% of the body weight (compared with that before the start of the experiment), and the tumor volume ≥1500 mm³, the experiment was terminated.

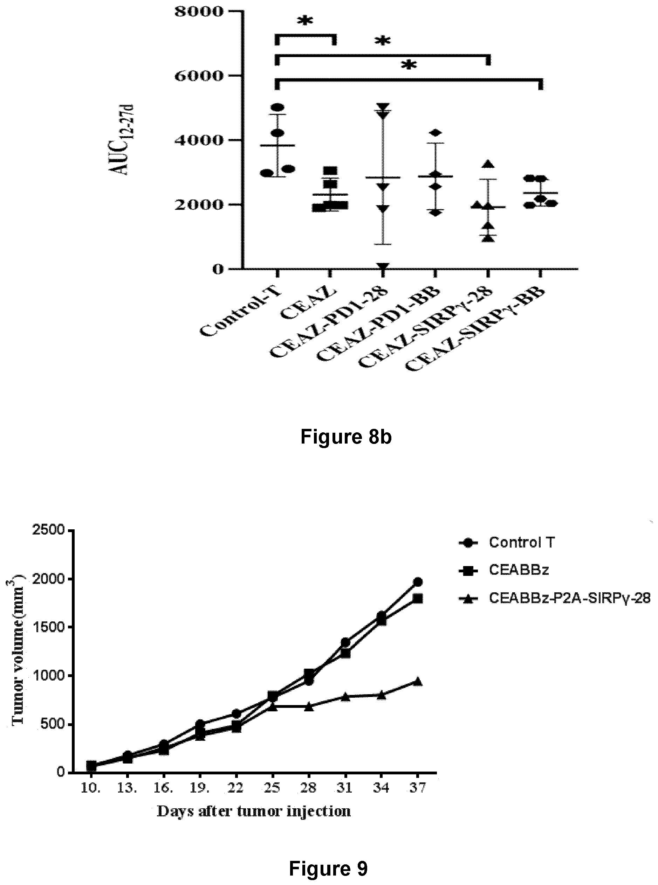

The tumor fluorescence value of mice was measured on Day 5 after tumor bearing, and the fluorescence value of *in vivo* imaging was used to randomly group the mice to ensure that there was no significant difference in the body weight and fluorescence value of the mice in each group, and the average body weight was calculated. On Day 6, CAR-T cells were re-infused in a volume of 100 µL (comprising 3×10⁶ effective CAR-T cells), and untransfected T cells with the same total cell number were given as a control group. The tumor volume growth curve was drawn based on tumor volume measurement data of NCG mice, and it was found that on Day 27-30, CEAZ-SIRPy-28 had a relatively obvious inhibitory effect on the tumor, and The results were shown in Figure 8b.

Further, the *in vivo* effectiveness of an immunosuppressive CAR (i.e., the CEABBZ-P2A-SIRPy-28 structure) designed by combining the second-generation CAR with the fusion protein was verified. NCG mice were also injected with DLD-1-CEA-Luc-GFP cells to bear tumors. After 13 days of tumor bearing, CAR-T cells were reinfused, and the total number of cells was 8×10⁶. The results were shown in FIG. 9 that the immunosuppressive CAR-T of the present application (CEABBZ-P2A-SIRPy-28) could function well *in vivo,* and the effect was obviously better than that of CEABBZ with the second-generation CAR structure of the control group.

Part II the experimental part of the CEA-targeting CAR structure (the amino acid sequence of CAR1 is SEQ ID NO: 27)

### Example 7 Construction of plasmid targeting CEA

### (1) Plasmid construction of experimental group

The hypoxia inducible promoter sequence 5HRE-CMVmini promoter was synthesized from the mini promoter miniCMV, and its nucleotide sequence was shown in SEQ ID NO: 1. Then the 5HRE-CMVmini promoter, lentiviral expression vector, CEAScFv-CD8 hinge region-CD8 transmembrane region-CD137-CD3ξ-P2A-SIRPγ-CD28 (5HCEA-BBZ-P2A-SIRPy-28) CAR structure were cleaved by double enzyme digestion and recovered, the gene fragments were connected and transformed, and single clones were picked, and the recombinant plasmid PBKL1-5H1P-CEA-OPRE (SIRPy fusion protein) comprising SIRPγ fusion protein of the CEA-targeting CAR-T cell preparation was constructed. In this step, the structure of our CAR comprising fusion protein can be obtained by two methods: the CAR and the fusion protein were co-expressed in one vector when transfecting immune cells or the CAR and the fusion protein were expressed separately in two vectors when transfecting immune cells. Wherein the CAR and the fusion protein were expressed separately in two vectors when transfecting immune cells to obtain the product abbreviated as "5HCEA-BBZ+SIRPγ-28".

### (2) Construction of plasmid in control group

5HCEA-BBZ-8H-8 was constructed according to the method of (1) in Example 1.

### Example 8 In vitro function validation of plasmid targeting CEA in models

5HCEA-BBZ-8H-8, SHCEA-BBZ-P2A-SIRPγ-28, SIRPy-28 plus CoCl2 were experimental verification hypoxia models. The results were shown in Figure 10 and Figure 11. The average fluorescence intensity and positive rate of 5HCEA-BBZ-8H-8, 5HCEA-BBZ-P2A-SIRPy-28, and SIRPy-28 are similar, as shown in Figure 12, 5HCEA-BBZ-P2A-SIRPy-28 had more advantages in amplification.

### Example 9 Validation of the effectiveness of CAR-T targeting CEA

### (1) Killing Efficiency of Target Cell DLDL1-CEA

CEA-positive DLD1-CEA and DLD1-CEA(CD47-) cells were used as target cells, respectively. After hypoxia treatment of effector cells (conventional CAR-T cells and CAR-T cells with Hypoxia inducible promoter CAR expression), they were plated on target cells according to the effector-target ratio of 1:1 and the killing ability of different CAR-Ts on target cells were detected.

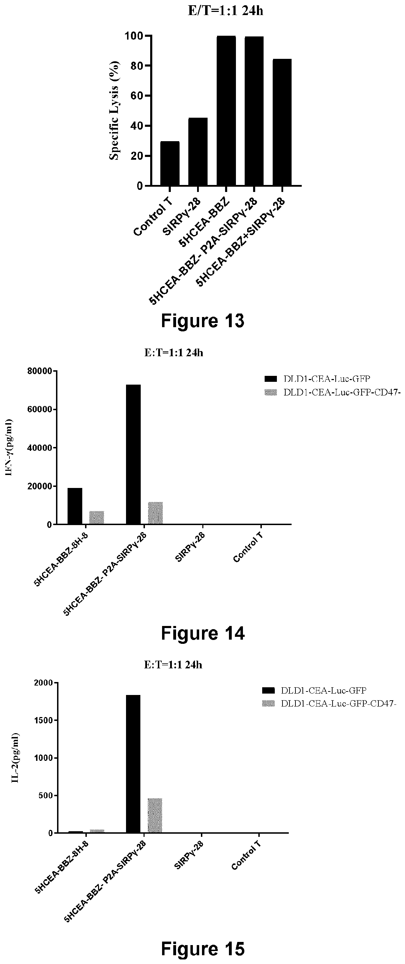

24h after adding CAR-T, the killing efficiency results of CAR-T on target cells DLDL1-CEA in each group are shown in Figure 13 and Table 4 below, 5HCEA-BBZ-8H-8, 5HCEA-BBZ+SIRPy-28 and 5HCEA-BBZ-P2A-SIRPy-28 had strong killing functions, and in the SIRPy-28 group, it also had a killing function.

**Table 4 The killing efficiency of each group of CAR-T targeting CEA on target cells DLDL1-CEA**

| Structure | Specific Lysis (%) |
|---|---|
| 5HCEA-BBZ+SIRPγ-28 | 84.4752 |
| 5HCEA-BBZ-P2A-SIRPγ-28 | 99.4934 |
| 5HCEA-BBZ-8H-8 | 99.7738 |
| SIRPγ | 45.2293 |
| Control T | 29.721 |
| Medium | 0 |

### (2) Detection of IFN-γ, IL-2, and TNF-α secretion

Followed by (1), the cell supernatant was collected 24 hours after killing, and the secretion capacity of IFN-γ, IL-2, and TNF-α was detected after CAR-T cells were stimulated by target cells. The collected supernatant was used to detect the secretion of IFN-γ and IL-2 by ELISA method using a kit.

The results were shown in Figure 14, Figure 15, and Figure 16. When the target cell was DLD1-CEA, in 5HCEA-BBZ-8H-8 group, IFN-γ, IL-2, and TNF-α were secreted less, and in 5HCEA-BBZ-P2A-SIRPy-28 group, IFN-γ, IL-2, and TNF-α were secreted much higher than that of 5HCEA-BBZ-8H-8 group, and when the target cells were DLD1-CEA (CD47-) cells, the secretion levels of IFN-γ, IL-2, and TNF-α were low or unable to reach the detection line. The 5HCEA-BBZ-P2A-SIRPy-28 of the present application is more conducive to the proliferation of CAR-T and the secretion of tumor-killing-related factors, indicating that it can indeed improve the effectiveness of CAR-T.

### Example 8 In vivo function verification of plasmids comprising SIRPγ fusion protein

DLD1-CEA-Luc-GFP cells were selected as the tumorigenic target cells used for *in vivo* validation to construct a human CEA+ solid tumor-bearing model.

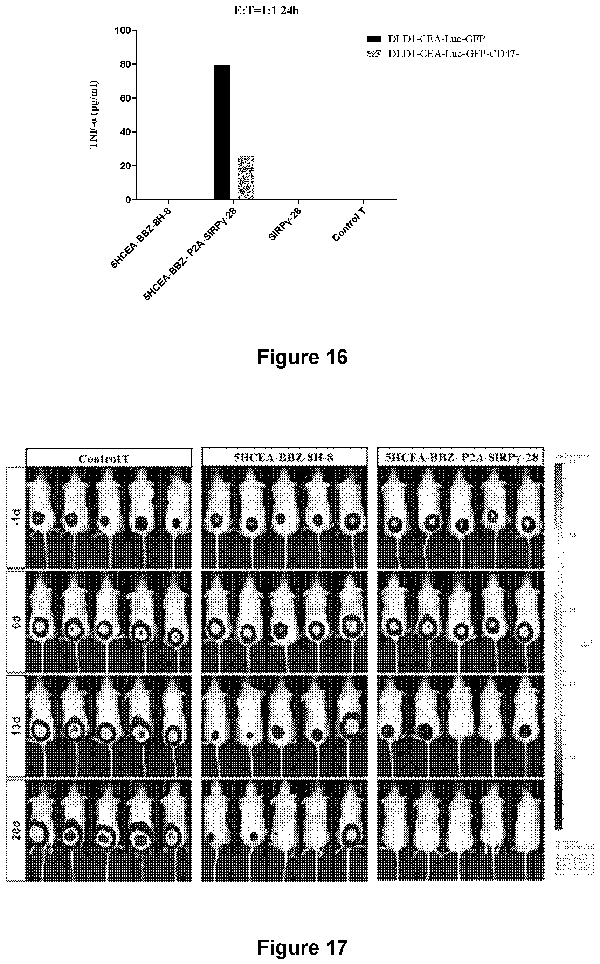

According to tumor volume, patients were randomly divided into Control T (CT) group, 5HCEA-BBZ-8H-8 group, 5HCEA-BBZ-P2A-SIRPy-28 group, and the control group was Control T group. On the 12th day of tumor formation, the corresponding CAR-T cells 1^{∗}10^7Copies/mice were injected into the tail vein of mice in different groups; the Control T group was infused with the same total number of T lymphocytes on the 12th day. The tumor volume of each group of mice was measured every three days. The experimental results were shown in Figure 17 and Figure 18. It could be seen that the *in vivo* effectiveness on mice of group 5HCEA-BBZ-8H-8 was significantly improved compared with that of group 5HCEA-BBZ-P2A-SIRPy-28, and in the 5HCEA-BBZ-8H-8 group, it also had a significant elimination effect on tumors.

### Part III Experimental part of the CAR structure targeting CD 19

### Example 9 Construction of plasmid targeting CD 19

Using SIRPγ and CD47 full-length plasmids, pL-CAG-2AGFP, pL-CAG-PD1-CD28-2ACherry, pL-CAG-PD1-BB-2ACherry as templates and a CD19-targeting CAR structure to construct vectors: SIRPy-28, 5HCD19-BBZ, and 5HCD19-BBZ-SIRPy-28. After verification by sequencing and alignment, the structures were successfully constructed.

### Example 10 Preparation of lentivirus and infection of T lymphocytes

The lentivirus was packaged in the calcium phosphate method to obtain three viral particles (SIRPγ-28, 5HCD19-BBZ, 5HCD19-BBZ-P2A-SIRPγ-28) in Example 1.

The lymphocytes were separated by gradient centrifugation. After centrifugation, the second layer (the white lymphocyte layer) was taken, washed with physiological saline, and cultured by adding RPMI 1640 complete medium comprising 10% FBS to obtain human PBMC cells. After the obtained PBMC cells were activated by anti-CD3 and CD28 monoclonal antibodies for 24 hours, the activated PBMCs were infected by a certain multiplicity of infection (MOI), and the positive rate of CAR-T was detected by flow cytometry on the 8th day of virus infection. The results were as shown in Figure 19 and Table 5 below.

**Table 5 The positive rate of CAR-T targeting CD19 in each experimental group detected by flow cytometry**

| Structure | % of CD3+ T Cells |
|---|---|
| Control T | 0.26 |
| SIRPγ-28 | 51.25 |
| 5HCD19-BBZ | 53.15 |
| 5HCD19-BBZ-P2A-SIRPγ-28 | 54.89 |
| SIRPγ-28+5HCD19-BBZ | 18.04 |

### Example 11 In vitro pharmacodynamic evaluation of CAR-T targeting CD19

Control T was used as the control group, and the experimental groups were set as SIRPy-28 group, 5HCD19-BBZ group, and 5HCD19-BBZ-P2A-SIRPγ-28 group. Nam6-Luc-GFP (CD19 positive) and K562-Luc-GFP (CD19 negative) were used as target cells, and the effectiveness was verified with *in vitro* killing and *in vitro* factor secretion. The results were shown in Figure 20 and Table 6 below, the *in vitro* killing effect of the product (5HCD19-BBZ-P2A-SIRPγ-28) obtained by transfecting immune cells with one co-expression vector and the product (SIRPγ-28+5HCD19-BBZ) obtained by co-transfecting immune cells with two separate expression vectors were significantly higher than that of the SIRPy-28 group and the 5HCD19-BBZ group, and they did not kill the negative cells.

As shown in Figure 21 and Table 7, the factor secretion of the product (5HCD19-BBZ-P2A-SIRPγ-28) obtained by transfecting immune cells with one co-expression vector and the product (SIRPγ-28+5HCD19-BBZ) obtained by co-transfecting immune cells with two separate expression vectors were significantly higher than that of SIRPy-28 group and 5HCD19-BBZ group.

**Table 6 Cell killing of each experimental group targeting CEA**

| Structure | Specific Lysis (%) |
|---|---|
| Control T | 37.2867 |
| SIRPγ-28 | 28.6995 |
| 5HCD19-BBZ | 84.3876 |
| 5HCD19-BBZ-P2A-SIRPγ-28 | 97.5187 |
| SIRPγ-28+5HCD19-BBZ | 97.4608 |

**Table 7 The secretion of IFN-γ factor in each experimental group targeting CEA**

| Structure | IFN-γ (pg/ml) |
|---|---|
| Control T | 135 |
| SIRPγ-28 | 453.97 |
| 5HCD19-BBZ | 17296.67 |
| 5HCD19-BBZ-P2A-SIRPγ-28 | 21820 |
| SIRPγ-28+5HCD19-BBZ | 14363.33 |

### Example 12 In vivo pharmacodynamic evaluation when targeting CD19

NCG mice (female, 6 weeks old) were selected and injected subcutaneously (s.c.) with Nalm6-Luc-GFP cells to establish an *in vivo* tumor-bearing model, and 8d after tumor-bearing, the dose of 1 × 10⁷ CAR-T Cells/mouse was injected into the tail vein (i.v.) to administer CAR-T to different groups (Control T, 5HCD19-BBZ, 5HCD19-BBZ-P2A-SIRPy-28). The tumor growth *in vivo* was observed by *in vivo* imaging, and the therapeutic effects of different CAR-Ts on lymphoma were evaluated *in vivo.* The results were shown in Figure 22 and Figure 23, and compared with the Control T group and the 5HCD19-BBZ group, 5HCD19-BBZ-P2A-SIRPγ-28 had significant anti-tumor effect *in vivo,* and could significantly eliminate the tumor.

### Part IV Experimental part of the CAR structure targeting PSCA

### Example 13 Construction of plasmid targeting PSCA and infection of T cells

### (1) Plasmid construction

The lentiviral expression vector and PSCA ScFv-G4H hinge region-CD28 transmembrane region-CD28-CD137-CD3ξ-P2A-SIRPγ-28 (PSCA-28BBZ-G4H-28TM-P2A-SIRPγ-28) CAR structure were cleaved by double enzyme digestion and recovered, the gene fragments were connected and transformed, and single clones were picked.

The lentivirus expression vector and PSCA ScFv-7H hinge region-CD28 transmembrane region-CD28-CD137-CD3ξ-P2A-SIRPγ-28 (PSCA-28BBZ-7H-28TM-P2A-SIRPγ-28) CAR structure were cleaved by double enzyme digestion and recovered, and the gene fragments were ligated and transformed and single clones were picked.

### (2) Infection of T cells

T cells were infected with the obtained plasmid to obtain CAR-T cells.

### Example 14 IFN-γ factor secretion of that targeting PSCA

Control T was used as the control group, and in the experimental groups, PSCA-28BBZ-G4H-28TM-P2A-SIRPγ-28, PSCA-28BBZ-7H-28TM-P2A-SIRPγ-28, PSCA-28BBZ-G4H-28TM+SIRPγ-28, PSCA-28BBZ-7H-28TM+SIRPγ-28, PSCA-28BBZ-G4H-28TM, and PSCA-28BBZ-7H-28TM were set as target cells, and RT4-Luc-GFP (PSCA positive) was the target cell, the *in vitro* effect was verified by *in vitro* factor secretion. The results were shown in Tables 8 to 13 and Figures 24 to 29. The IFN-γ factor secretions of the products PSCA-28BBZ-G4H-28TM-P2A-SIRPγ-28 or PSCA-28BBZ-7H-28TM-P2A-SIRPγ-28 obtained by transfecting immune cells with one co-expression vector and the products PSCA-28BBZ-G4H-28TM+SIRPγ-28 or PSCA-28BBZ-7H-28TM+SIRPγ-28 obtained by co-transfecting immune cells with two separate expression vectors were significantly higher than that of the control group and PSCA-28BBZ-G4H-28TM group or PSCA-28BBZ-7H-28TM group and, and they did not kill the negative cells.

**Table 8 PSCA-28BBZ-G4H-28TM-P2A-SIRPγ-28 secreted IFN-γ factor**

| Structure | IFN-γ (pg/ml) |
|---|---|
| PSCA-28BBZ-G4H-28TM-P2A-SIRPγ-28 | 4060.33 |
| Control T | 234.23 |

**Table 9 PSCA-28BBZ-7H-28TM-P2A-SIRP-28 secreted IFN-γ factor**

| Structure | IFN-γ (pg/ml) |
|---|---|
| PSCA-28BBZ-7H-28TM-P2A-SIRPγ-28 | 4024.67 |
| Control T | 234.23 |

**Table 10 PSCA-28BBZ-G4H-28TM+SIRPγ-28 secreted IFN-γ factor**

| Structure | IFN-γ (pg/ml) |
|---|---|
| PSCA-28BBZ-G4H-28TM+SIRPγ-28 | 6862.33 |
| Control T | 234.23 |

**Table 11 PSCA-28BBZ-7H-28TM+SIRP-28 secreted IFN-γ factor**

| Structure | IFN-γ (pg/ml) |
|---|---|
| PSCA-28BBZ-7H-28TM+SIRPγ-28 | 8550.67 |
| Control T | 234.23 |

**Table 12 PSCA-28BBZ-G4H-28TM, PSCA-28BBZ-G4H-28TM+SIRPγ-28 secreted IFN-γ factor**

| Structure | IFN-γ (pg/ml) |
|---|---|
| PSCA-28BBZ-G4H-28TM | 6998.67 |
| PSCA-28BBZ-G4H-28TM+SIRPγ-28 | 6862.33 |
| Control T | 234.23 |

**Table 13 PSCA-28BBZ-7H-28TM, PSCA-28BBZ-7H-28TM+SIRPγ-28 secreted IFN-γ factor**

| Structure | IFN-γ (pg/ml) |
|---|---|
| PSCA-28BBZ-7H-28TM | 6660.33 |
| PSCA-28BBZ-7H-28TM+SIRPγ-28 | 8550.67 |
| Control T | 234.23 |

### Example 14 Cell killing of that targeting PSCA

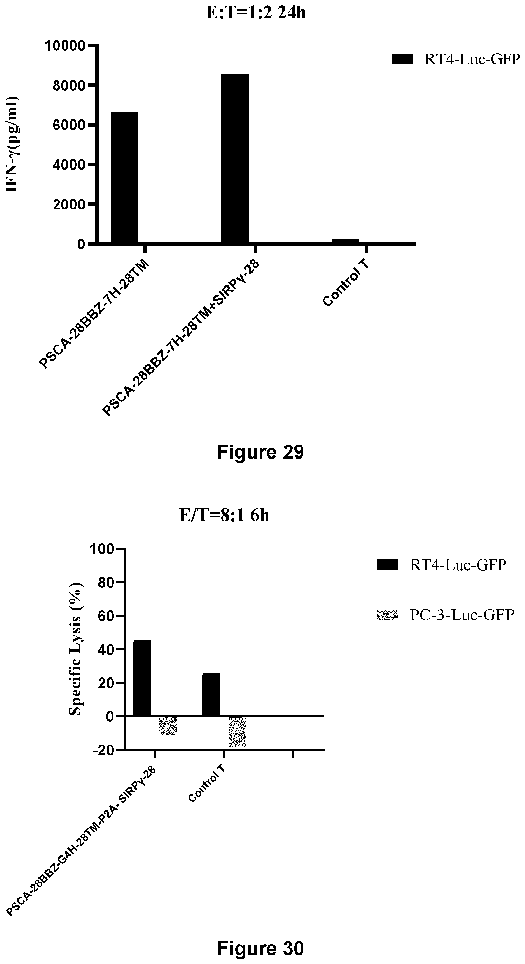

The experimental groups were set up with PSCA-28BBZ-G4H-28TM-P2A-SIRPy-28, PSCA-28BBZ-7H-28TM-P2A-SIRPγ-28, PSCA-28BBZ-G4H-28TM+SIRPγ-28, PSCA-28BBZ-7H-28TM+SIRPy-28, PSCA-28BBZ-G4H-28TM, PSCA-28BBZ-7H-28TM, and using RT4-Luc-GFP (PSCA positive) and PC-3-Luc-GFP (PSCA negative) as target cells respectively. The results were shown in Table 14-Table 19 and Figure 30-Figure 35. The *in vitro* killing of the product PSCA-28BBZ-G4H-28TM-P2A-SIRPγ-28 or PSCA-28BBZ-7H-28TM-P2A-SIRPγ-28 obtained by transfecting immune cells with one co-expression vector and the product PSCA-28BBZ-G4H-28TM+SIRPγ-28 or PSCA-28BBZ-7H-28TM+SIRPγ-28 obtained by co-transfecting immune cells with two separate expression vectors were significantly higher than that of the control group and PSCA-28BBZ-G4H-28TM group or PSCA-28BBZ-7H-28TM group, and they did not kill the negative cells.

**Table 14 Cell killing of PSCA-28BBZ-G4H-28TM-P2A-SIRPγ-28 in positive/negative cells, respectively**

| Structure | IFN-γ (pg/ml) | |
|---|---|---|
| | RT4-Luc-GFP | PC-3-Luc-GFP |
| PSCA-28BBZ-G4H-28TM-P2A-SIRPγ-28 | 45.2911 | -0.7548 |
| Control T | 25.6632 | -8.3602 |

**Table 15 Cell killing of PSCA-28BBZ-7H-28TM-P2A-SIRPγ-28 in positive/negative cells, respectively**

| Structure | IFN-γ (pg/ml) | |
|---|---|---|
| | RT4-Luc-GFP | PC-3-Luc-GFP |
| PSCA-28BBZ-7H-28TM-P2A-SIRPγ-28 | 46.2139 | -2.4081 |
| Control T | 25.6623 | -8.3602 |

**Table 16 Cell killing of PSCA-28BBZ-G4H-28TM+SIRPγ-28 in positive/negative cells, respectively**

| Structure | IFN-γ (pg/ml) | |
|---|---|---|
| | RT4-Luc-GFP | PC-3-Luc-GFP |
| PSCA-28BBZ-G4H-28TM+SIRPγ-28 | 69.9491 | -9.91984 |
| Control T | 25.6632 | -18.3602 |

**Table 17 Cell killing of PSCA-28BBZ-7H-28TM+SIRPγ-28 in positive/negative cells, respectively**

| Structure | IFN-γ (pg/ml) | |
|---|---|---|
| | RT4-Luc-GFP | PC-3-Luc-GFP |
| PSCA-28BBZ-7H-28TM+SIRPγ-28 | 76.6439 | -10.0736 |
| Control T | 25.6632 | -18.3602 |

**Table 18 Cell killing in PSCA-28BBZ-G4H-28TM and PSCA-28BBZ-G4H-28TM+SIRPγ-28**

| Structure | IFN-γ (pg/ml) | |
|---|---|---|
| | RT4-Luc-GFP | PC-3-Luc-GFP |
| PSCA-28BBZ-G4H-28TM | 53.5258 | -19.8898 |
| PSCA-28BBZ-G4H-28TM+SIRPγ-28 | 69.9491 | -9.91984 |
| Control T | 25.6632 | -18.3602 |

**Table 19 Cell killing in PSCA-28BBZ-7H-28TM, PSCA-28BBZ-7H-28TM+SIRPγ-28**

| Structure | IFN-γ (pg/ml) | |
|---|---|---|
| | RT4-Luc-GFP | PC-3-Luc-GFP |
| PSCA-28BBZ-7H-28TM | 52.6503 | -12.1743 |
| PSCA-28BBZ-7H-28TM+SIRPγ-28 | 76.6439 | -10.0736 |
| Control T | 25.6632 | -18.3602 |

Finally, it should be noted that the above examples are only used to illustrate the technical solutions of the present application and not to limit them. Although the present application has been described in detail with reference to the preferred examples, those of ordinary skills in the art should understand that modifications or equivalent substitutions can be made to the technical embodiments of the present application without departing from the spirit and scope of the technical embodiments of the present application and should be included in the scope of the claims of the present application.

## Claims

1. A fusion protein for reversing the tumor microenvironment, wherein the fusion protein is a SIRPγ fusion protein, and the structure of the SIRPγ fusion protein comprises an extracellular domain, a transmembrane domain and an intracellular signaling domain.

2. The fusion protein according to claim 1, wherein the transmembrane domain is derived from human CD28 transmembrane domain or human CD8 transmembrane domain, preferably, the intracellular signaling domain is derived from CD28 or 4-1BB.

3. The fusion protein according to claim 2, wherein the amino acid sequence of the transmembrane domain is shown in SEQ ID NO:7 or SEQ ID NO:8; preferably, the intracellular signaling domain is derived from CD28, and the sequence of the intracellular signaling domain is shown in SEQ ID NO:9 or SEQ ID NO:38.

4. The fusion protein according to claim 1, wherein the structure of the SIRPγ fusion protein is SIRPy-CD28TM-CD28 or SIRPγ-CD8TM-4-1BB.

5. The fusion protein according to claim 1, wherein the amino acid sequence of the extracellular domain of SIRPγ is shown in SEQ ID NO: 1 or is a functional variant thereof.

6. The fusion protein according to claim 4, wherein the amino acid sequence of the SIRPγ fusion protein SIRPy-CD28TM-CD28 is shown in SEQ ID NO: 2 or is a functional variant thereof.

7. The fusion protein according to claim 4, wherein the amino acid sequence of the SIRPγ fusion protein SIRPγ-CD8TM-4-1BB is shown in SEQ ID NO: 3 or is a functional variant thereof.

8. The fusion protein according to claim 6, wherein the nucleotide sequence of the SIRPγ fusion protein SIRPy-CD28TM-CD28 is shown in SEQ ID NO: 13.

9. The fusion protein according to claim 7, wherein the nucleotide sequence of the SIRPγ fusion protein SIRPγ-CD8TM-4-1BB is shown in SEQ ID NO: 14.

10. An expression vector comprising the fusion protein according to any one of claims 1-9.

11. The expression vector according to claim 10, wherein the expression vector is any one of a lentivirus expression vector, a retrovirus expression vector, an adenovirus expression vector, an adeno-associated virus expression vector, a DNA vector, an RNA vector, and a plasmid.

12. An immune cell comprising the expression vector according to any one of claims 10-11.

13. The immune cell according to claim 12, wherein the immune cell is a T cell, a T cell precursor or an NK cell.

14. The immune cell according to claim 13, wherein it comprises a structure with a chimeric antigen receptor that recognizes a tumor antigen, the chimeric antigen receptor comprises an extracellular domain recognizing a tumor antigen, a hinge region, a transmembrane domain and an intracellular signaling domain, the tumor antigen include but is not limited to PSCA, PSMA, CD19, BCMA, CD123, CD20, CD22, CEA, EGFR, EGFRVIII, GPC3, 5T4, CD33, Her2, GD2, CD70, CLL-1, Trop2, CD47, GPC3, CLND18.2, CD133, CS1, CD155, CD30, ROR1, MUC1, IL13RAα2 or mesothelin that can be used as an antigenic molecule for tumor targeting recognition.

15. A tumor immunosuppression-resistant CAR, wherein the CAR comprises the fusion protein according to any one of claims 1-9 and CAR1, and the CAR1 comprises an extracellular domain recognizing a tumor antigen, a hinge region, a transmembrane domain and an intracellular signaling domain.

16. The tumor immunosuppression-resistant CAR according to claim 15, wherein the fusion protein is linked to CAR1 through a polycistronic structure, and the polycistronic structure is a self-cleaving polypeptide or an internal ribosome entry site IRES, and the self-cleaving polypeptide is T2A, P2A, E2A or F2A.

17. The tumor immunosuppression-resistant CAR according to claim 15, wherein the CAR structure is an ScFv-hinge-TM-CD3ζ-self-cleaving peptide-SIRPy fusion protein or an ScFv-hinge-TM-4-1BB-CD3ζ-self-cleaving peptide-SIRPy fusion protein.

18. The tumor immunosuppression-resistant CAR according to claim 15, wherein the CAR structure is an ScFv-hinge-TM-CD3ζ-self-cleaving peptide-SIRPγ-CD28TM-CD28 or an ScFv-hinge-TM-4-1BB-CD3ζ-self-cleaving peptide-SIRPy-CD28TM-CD28.

19. The tumor immunosuppression-resistant CAR according to claim 15, wherein in the CAR1 structure: the amino acid sequence of the hinge is shown in SEQ ID NO: 24 or is a functional variant thereof, and the amino acid sequence of the TM is shown in SEQ ID NO: 7 or SEQ ID NO: 8, the amino acid sequence of CD3ζ is shown in SEQ ID NO: 11 or is a functional variant thereof; and in the structure of the fusion protein: the amino acid sequence of the extracellular domain of SIRPγ is shown in SEQ ID NO: 1 or is a functional variant thereof; the amino acid sequence of the transmembrane domain derived from human CD28 is shown in SEQ ID NO:7; the amino acid sequence of the intracellular signaling domain derived from human CD28 is shown in SEQ ID NO:9.

20. The tumor immunosuppression-resistant CAR according to any one of claims 17-19, wherein the ScFv recognizes any one or more of CD19, CD123, MOv-y, PSMA, IL13Rα2, EGFRvIII, EGFR, EPCAM, GD2, MUC1, HER2, GPC3, CEA, Meso, CD133, NKG2D, CD138, LeY, k-Light, CD33, ROR1, BCMA, CD30, CD20, CD22, PSCA, CLL-1, CD70, and CD47.

21. The tumor immunosuppression-resistant CAR according to claim 20, wherein the amino acid sequence of the ScFv is shown in SEQ ID NO: 25 or is a functional variant thereof.

22. The tumor immunosuppression-resistant CAR according to claim 15 or 16, wherein the CAR1 comprises one of the following:
a) the CAR1 comprising a CEA single chain antibody, a CD8 hinge region, a CD8 transmembrane domain, a CD137-CD3ξ dual-stimulus signal; preferably, the amino acid sequence of the CAR structure is shown in SEQ ID NO: 26 or is a functional variant thereof;
or b) the CAR1 comprising a CD19 single chain antibody, a CD8 hinge region, a CD8 transmembrane domain, a CD137-CD3ξ dual-stimulus signal; preferably, the amino acid sequence of the CAR structure is shown in SEQ ID NO: 27 or is a functional variant thereof;
or c) the CAR1 comprising a PSCA single-chain antibody, a hinge region, a CD28 transmembrane domain, a CD28-CD137-CD3ξ tristimulus signal; the hinge region is G4H or 7H; preferably, the amino acid sequence of the CAR structure is shown in SEQ ID NO: 28 or is a functional variant thereof; or is shown in SEQ ID NO: 29 or is a functional variant thereof.

23. The tumor immunosuppression-resistant CAR according to claim 22, wherein the CAR1 structures in a) and b) further comprise a hypoxia inducible promoter, and the nucleic acid sequence of the hypoxia inducible promoter comprises the sequence shown in SEQ ID NO:30.

24. A nucleic acid sequence, wherein it encodes the tumor immunosuppression-resistant CAR according to claim 21 or 22, the nucleic acid sequence comprises the sequence shown in SEQ ID NO: 31, SEQ ID NO: 32, SEQ ID NO: 33, SEQ ID NO: 34 or SEQ ID NO: 35.

25. An expression vector comprising the tumor immunosuppression-resistant CAR according to any one of claims 15-23, wherein the expression vector is any one of a lentivirus expression vector, a retrovirus expression vector, an adenovirus expression vector, an adeno-associated virus expression vector, DNA vector, RNA vector and plasmid.

26. An immune cell comprising the expression vector according to claim 25.

27. The immune cell according to claim 26, wherein the immune cell is a T cell, a T cell precursor or an NK cell.

28. A method for preparing the immune cell according to claim 14, wherein the CAR structure not comprising the SIRPγ fusion protein and the SIRPγ fusion protein are co-expressed in a vector when transfecting immune cells; or the CAR structure not comprising the SIRPγ fusion protein and the SIRPγ fusion protein are expressed separately in two vectors when transfecting immune cells.

29. A pharmaceutical composition, wherein the pharmaceutical composition comprises the fusion protein according to any one of claims 1-9 or the tumor immunosuppression-resistant CAR according to any one of claims 15-23 or the immune cell according to any one of claims 12-14 or the immune cell according to any one of claims 26-27 or the expression vector according to any one of claims 10-11 or the expression vector according to claim 25 or the nucleic acid sequence according to claim 24.

30. The pharmaceutical composition according to claim 19, wherein the pharmaceutical composition further comprises an active agent that can enhance the expression activity of the CAR and/or a therapeutic agent.

31. The use of the pharmaceutical composition of claim 29 or 30 in the preparation of medicaments of tumors.

32. The use according to claim 31, wherein the tumors are malignant tumors, including acute lymphoid leukemia, chronic lymphocytic leukemia, chronic myeloid leukemia, non-Hodgkin's lymphoma, Hodgkin's lymphoma, prostate cancer, colorectal cancer, breast cancer, ovarian cancer, cervical cancer, pancreatic cancer, lung cancer, kidney cancer, liver cancer, brain cancer and skin cancer, the tumors highly express any one or more of CD19, CD123, MOv-y, PSMA, IL13Rα2, EGFRvIII, EGFR, EPCAM, GD2, MUC1, HER2, GPC3, CEA, Meso, CD133, NKG2D, CD138, LeY, k-Light, CD33, ROR1, BCMA, CD30, CD20, CD22, PSCA, CLL-1, CD70, and CD47.
